(19)

**Europäisches Patentamt**
**European Patent Office**
**Office européen des brevets**

(11) **EP 2 124 798 B1**

(12) **EUROPEAN PATENT SPECIFICATION**

(45) Date of publication and mention
of the grant of the patent:
**10.04.2019 Bulletin 2019/15**

(21) Application number: **08728601.9**

(22) Date of filing: **30.01.2008**

(51) Int Cl.:
*A61M 25/01* (2006.01)  *A61B 17/00* (2006.01)
*A61B 34/00* (2016.01)  *A61B 34/30* (2016.01)
*A61B 34/37* (2016.01)  *A61B 34/10* (2016.01)

(86) International application number:
**PCT/US2008/052516**

(87) International publication number:
**WO 2008/095032 (07.08.2008 Gazette 2008/32)**

(54) **ROBOTIC INSTRUMENT SYSTEMS CONTROLLED USING KINEMATICS AND MECHANICS MODELS**

DURCH KINEMATIK- UND MECHANIKMODELLE GESTEUERTE
ROBOTERINSTRUMENTENSYSTEME

SYSTEMES D'INSTRUMENTS ROBOTISES COMMANDES UTILISANT DES MODELES
CINEMATIQUES ET MECANIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **30.01.2007   US 898661 P**

(43) Date of publication of application:
**02.12.2009   Bulletin 2009/49**

(73) Proprietor: **Auris Health, Inc.
Redwood City, CA 94065 (US)**

(72) Inventor: **CAMARILLO, David
Aptos, CA 95003 (US)**

(74) Representative: **Lecomte & Partners
P.O. Box 1623
1016 Luxembourg (LU)**

(56) References cited:
**EP-A- 1 250 986        WO-A-2004/029782
WO-A-2005/087128**

• **WAYNE LAWTON, RAGHU RAGHAVAN, S R
RANJAN, RAJU VISWANATHAN: "Ribbons and
groups: a thin rod theory for catheters and
filaments" JOURNAL OF PHYSICS A, vol. 32, no.
9, 1999, pages 1709-1735, XP002490480 UK**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates generally to robotically controlled systems, and more particularly, to control systems for manipulating robotic catheter systems used in minimally invasive diagnostic and therapeutic procedures.

BACKGROUND

Robotic interventional systems and devices are well suited for use in performing minimally invasive medical procedures, as opposed to conventional techniques wherein the patient's body cavity is open to permit the surgeon's hands access to internal organs. Such robotic systems are useful to facilitate imaging, diagnosis, and treatment of tissues which may lie deep within a patient, and which may be accessed via naturally-occurring pathways such as blood vessels, other lumens, via surgically-created wounds of minimized size, or combinations thereof.

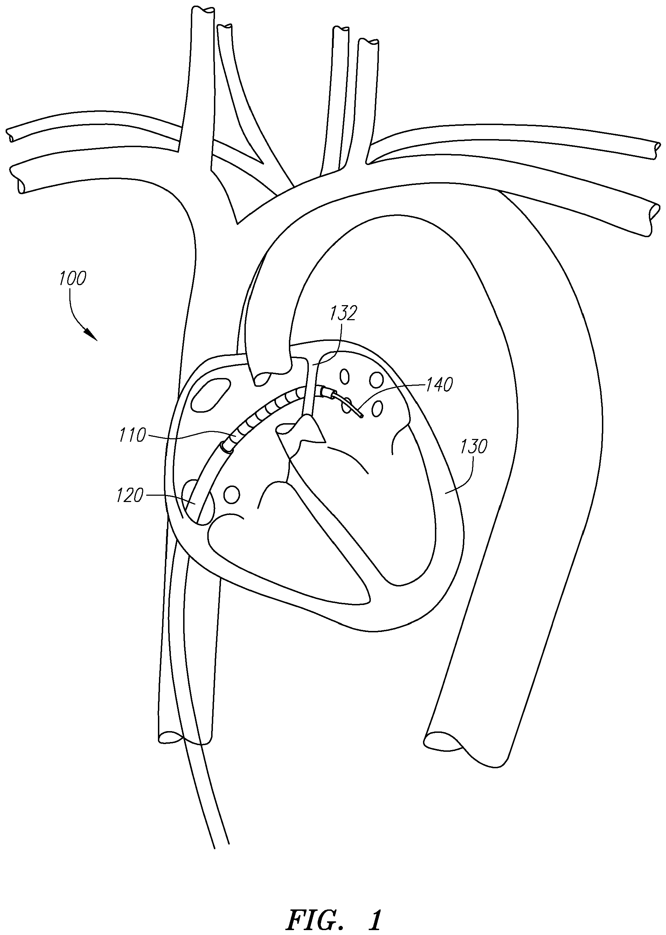

Figure 1 illustrates one example of how robotic intravascular systems may be utilized to position a catheter or other working instrument within the heart, e.g., to treat or ablate endocardial tissue. In the illustrated example, a robotically controlled instrument 100 including a catheter or guide instrument 110 and a sheath instrument 120 is positioned within the heart 130. Figure 1 depicts delivery of the instrument 100 utilizing a standard atrial approach in which the robotically controlled catheter 110 and sheath 120 pass through the inferior vena cava and into the right atrium. An imaging device, such as an intracardiac echo ("ICE") sonography catheter (not shown in Figure 1), may be forwarded into the right atrium to provide a field of view upon the interatrial septum. The catheter 110 may be driven to the septum wall, and the septum 132 may be crossed using a conventional technique of first puncturing the fossa ovalis location with a sharpened device, such as a needle or wire, passed through a working lumen of the catheter 110, then passing a dilator 140 over the sharpened device and withdrawing the sharpened device to leave the dilator 140, over which the catheter 110 may be advanced. Various working instruments (not shown in Figure 1) may be delivered through the lumen of the catheter 110 as necessary and depending on the surgical application. For example, the working instrument may be an ablation catheter that delivers targeted radio frequency (RF) energy to selected endocardial tissue, e.g., to treat atrial fibrillation. Further aspects of such systems, devices and applications are described in U.S. Publication No. 2006/0100610 A1.

Such robotic instrument systems typically account for certain types of catheter motion or device attributes using a kinematics model. A "kinematics" model is related to the motion and shape of an instrument, without consideration of forces on the instrument that bring about that motion. In other words, a kinematics model is based on geometric parameters and how a position of the instrument changes relative to a pre-determined or reference position or set of coordinates. One example of a kinematics model that may be used in non-invasive robotic applications receives as an input a desired or selected position of the instrument, e.g., a position within the heart, and outputs a corresponding shape or configuration of the instrument, e.g., with reference to a current or known shape or configuration, that results in positioning of the instrument according to the input.

While known systems have been utilized with success, the manner in which components are controlled can be improved. For example, kinematics control models used in known systems do not account for forces on a catheter or other elongate instrument that bring about motion or bending of the instrument. For example, bending or positioning of an instrument may compress or stretch the instrument. Known kinematics control models, however, do not account for these compression and stretching forces, thereby resulting in potential errors. Such compression forces may also result in slack deflection members, thereby potentially reducing the accuracy and ability to control bending and positioning of the instrument. Further, known kinematics control models may result in certain deflection member forces that are not minimized or optimized. WO 2005/087128 A discloses a robotic instrument system according to the preamble of claim 1.

SUMMARY

The invention relates to a robotic instrument system according to claim 1. According to one example a robotic instrument system comprises a catheter, a controller and an actuator. The catheter includes a deflection member. The actuator is operably coupled to the controller and the deflection member. The controller is configured to execute a control model comprising a kinematics model and a mechanics model, and control the actuator based the kinematics and mechanicals models such that the catheter may bend when the deflection member is moved by the actuator.

Another example is directed to a robotic instrument system comprising an elongate instrument, such as a catheter, comprising a pull wire, a controller and a servo motor. The controller includes a control model having a kinematics model and a static mechanics model, and the servo motor operably coupled to the controller and the pull wire. The controller is configured to control the servo motor based on serial execution of the kinematics and mechanicals models such that the catheter may bend when tension on the pull wire is changed by actuation of the servo motor based on an output of the mechanics model.

Also disclosed is a method of controlling a robotic surgical instrument system. The method comprises executing a control model of a catheter including a deflection member, the control model comprising a kinematics model and a mechanics model of the catheter; and controlling an actuator based on the kinematics and mechanics models to move the deflection member and bend the catheter.

According to another example a robotic instrument system comprises an elongate catheter instrument, an elongate sheath instrument, servo motors, and a controller. The catheter instrument includes a distal bending portion and a deflection member, and the elongate sheath instrument includes a distal bending portion and a deflection member. The catheter instrument is carried coaxially in the sheath instrument, and the distal bending portion of the catheter instrument can be moved to extend out of, and retract into, respectively, a distal opening of the sheath instrument. The controller is configured to control actuation of a servo motor associated with the guide instrument based on a catheter control model comprising a kinematics model and a mechanics model to controllably displace the catheter instrument deflection member and bend the catheter instrument distal bending portion. The controller is also configured to control actuation of a servo motor associated with the sheath instrument to controllably displace the sheath instrument deflection member and bend the sheath instrument distal bending portion.

Also disclosed is a method of controlling a robotic surgical instrument system. The method comprises executing a control model of an elongate catheter instrument including a deflection member, the catheter instrument control model comprising a kinematics model of the guide instrument and a mechanics model of the guide instrument, and executing a control model of an elongate sheath instrument including a deflection member. The method further comprises controlling actuation of a servo motor associated with the catheter instrument in response to the catheter instrument control model to move the catheter instrument deflection member and bend a distal portion of the catheter instrument, and controlling actuation of a servo motor associated with the sheath instrument in response to the sheath instrument control model to move the sheath instrument deflection member and bend a distal portion of the sheath instrument.

In accordance with a further example a robotic instrument system comprises an elongate instrument having a deflection member, a controller and an actuator operably coupled to the controller and the deflection member. The controller includes a mechanics control model and is configured to control the actuator based the mechanics model such that the elongate instrument may bend when the deflection member is moved by the actuator.

Also disclosed is a method of controlling a robotic surgical instrument system and comprises executing a mechanics control model of a catheter including a deflection member; and controlling actuation of an actuator based on the mechanics model to move the deflection member and bend the catheter.

According to yet another example, a robotic instrument system comprises a catheter comprising a deflection member, a controller and an actuator operably coupled to the controller and the deflection member. The controller is configured to execute a control model comprising a first model and a second model different than the first model, at least one of the first and second models accounting for a force on the elongate instrument, and further configured to control the actuator based the first and second models such that the catheter may bend when the deflection member is moved by the actuator.

Also disclosed is a method of controlling a robotic surgical instrument system and comprises executing a control model of a catheter including a deflection member, the control model comprising a first model and a second model different than the first model, at least one of the first and second models accounting for a force on the catheter; and controlling an actuator based on the first and second models to move the deflection member and bend the catheter.

In one or more examples, the elongate instrument is a guide instrument or a catheter, the actuator is a servo motor, and the deflection member is a wire. In one or more examples involving different models, a control model includes a kinematics model and a mechanics model. The kinematics and mechanics models are based on different variables and executed serially by the controller. An input, which may be filtered to maintain positive deflection member tension, is provided to the kinematics model, which generates an output or result, which is provided as an input to the mechanics model. The output of the mechanics model controls actuation of the actuator, thereby controlling movement or pulling on the deflection member and bending of the elongate instrument.

In one or more examples, the kinematics model input comprises a position of the elongate guide instrument, the kinematics model output comprises a configuration or shape of the elongate guide instrument corresponding to the position of the elongate guide instrument, and the mechanics model output comprises a deflection member displacement. In this manner, the kinematics model, which does not account for force on the elongate guide, generates an output that does not directly actuate the actuator to move the deflection member, in contrast to known systems that use only a kinematics model.

In one or more examples, the mechanics model, which does take into account force and may be based on forces applied to a continuous deflection member extending through the elongate guide represented as a beam, may be expressed as

$$\Delta l_t = l_0 \left( \mathbf{G}^T + \frac{1}{K_t} \mathbf{G}^\dagger \mathbf{K}_m \right) \mathbf{q}.$$

Wherein $\Delta lt$ = a displacement of the deflection member resulting from actuation of the actuator, $l_o$ = length of the deflection member, G = a geometric representation of the deflection member in the form of a matrix, $G^T$ = transpose of G, $G^\dagger$ = is the inverse of G, $K_t$ = stiffness of the deflection member, $K_m$ = stiffness of the elongate instrument, and q= an output of the kinematics model representing a configuration or shape of the elongate instrument. The mechanics model may be a linear model such that tension of the deflection member or wire is linearly related to a radius of bending of the elongate instrument and may account for multiple, e.g., four, deflection members. In this manner, the control model accounts for curvature of the elongate guide instrument and compression on the elongate guide instrument.

In one or more examples involving catheter and sheath instruments, bending of the catheter may be controlled using both kinematics and mechanics models of the catheter, whereas bending of the sheath instrument may be controlled using a kinematics model. Further, bending of the sheath instrument may be controlled using both kinematics and mechanics models.

BRIEF DESCRIPTION OF THE DRAWINGS

Referring now to the drawings in which like reference numbers represent corresponding parts throughout and in which:

Figure 1 generally illustrates one application of a robotically controlled guide instrument to position a catheter instrument within the heart;
Figure 2 schematically illustrates a system constructed according to one embodiment that includes a controller having kinematics and non-kinematics models;
Figure 3 is a flow chart of a method for controlling a deflection member to control an instrument using the system illustrated in Figure 2;
Figure 4A generally illustrates a steerable cardiac catheter;
Figure 4B is a cross-sectional view of a steerable catheter carrying a working instrument;
Figure 5 schematically illustrates a system constructed according to one embodiment that includes a controller having kinematics and mechanics models;
Figure 6 is a flow chart of a method for controlling a deflection member to control an instrument using the system illustrated in Figure 2;
Figure 7 schematically illustrates inputs and outputs of a kinematics model for use in embodiments;
Figure 8 schematically illustrates inputs and outputs of a mechanics model for use in embodiments;
Figure 9A illustrates a catheter instrument having a distal bending portion;
Figure 9B is a cross-sectional view of the catheter instrument illustrated in Figure 9A having a plurality of deflection members;
Figure 10 illustrates an instrument having a catheter that may be controlled using mechanics model embodiments by controlling one or more tension members using an instrument driver;
Figure 11 illustrates one example of an instrument driver that may be used with embodiments;
Figure 12 is a more detailed illustration of one example of an instrument driver that may be used with embodiments;
Figure 13 schematically illustrates a robotically controlled system in which embodiments of the invention may be implemented;
Figure 14 illustrates an instrument modeled as a cantilever beam deflected by a deflection member and a differential segment thereof;
Figure 15 is a free body diagram for a beam that is used to establish a model for a mechanics model according to one embodiment;
Figure 16 is a free body diagram of a deflection member that is used to establish a model for a mechanics model according to one embodiment;
Figure 17 illustrates strain fields from bending and compression with a resulting neutral axis;
Figure 18 illustrates a spring model for a deflection member-catheter manipulator system;
Figure 19 is a block diagram for model information flow according to one embodiment;
Figure 20 schematically illustrates a system constructed according to one embodiment that utilizes the configuration shown in Figure 19 and kinematics and mechanics models;
Figure 21 illustrates an input filter constructed according to one embodiment;
Figure 22 illustrates a simulated three-deflection member catheter manipulator;
Figure 23A illustrates a minimum-norm solution for three deflection members;

Figure 23B illustrates a mini-max solution for three deflection members;

Figure 23C illustrates a unique solution for two deflection members;

Figure 23D illustrates a minmax solution for four deflection members;

Figures 24A-D illustrate single deflection member data points obtained by flexing or compression (diamonds) and extension (crosses), wherein Figure 24A illustrates a configured utilized during an experiment, Figure 24B illustrates bend curve measurements in comparison to constant curvature (solid line); Figure 24C illustrates axial linearity with a resulting average stiffness, and Fig. 24D illustrates bending linearity with a resulting average stiffness;

25A illustrates a comparison of commanded values (solid line) versus measured values (diamonds) for axial compression; and

Figure 25B illustrates a comparison of commanded values (solid line) versus measured values (diamonds) for curvature.

## DETAILED DESCRIPTION OF ILLUSTRATED EMBODIMENTS

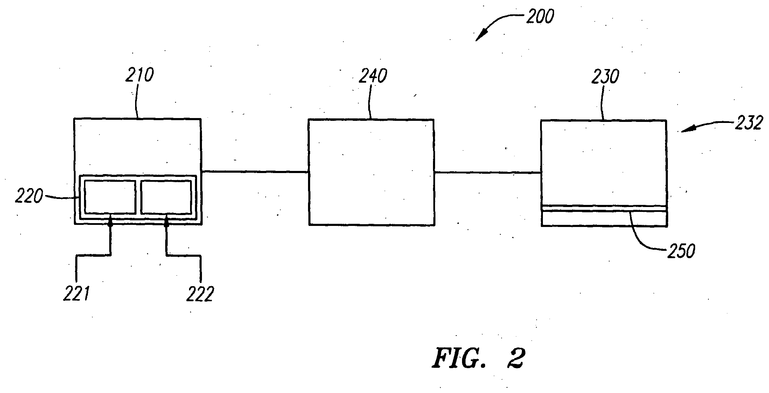

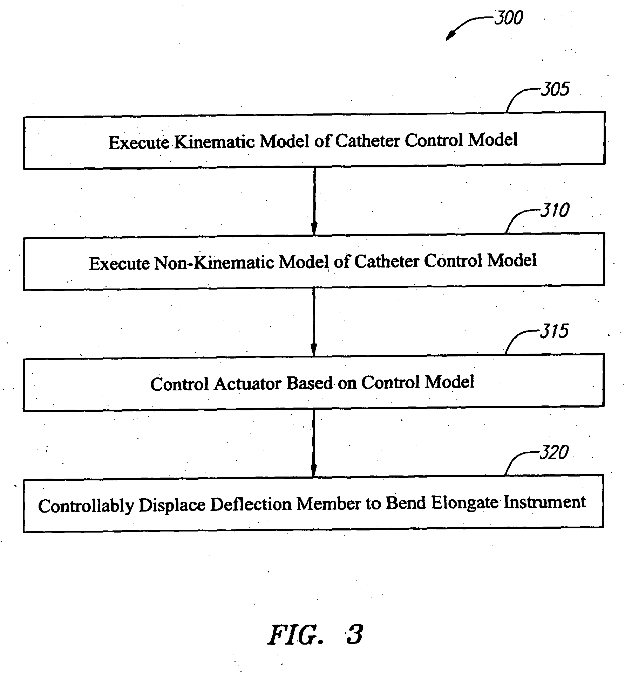

Referring to Figure 2, and with further reference to Figure 3, system 200 and method 300 advantageously provide a controller 210 that is programmed or otherwise configured with appropriate hardware and/or software to execute a multi-model control model 220. In the illustrated example, the control model 220 includes two different models or model components 221, 222 (generally referred to as models 221, 222). The model 221 is a "kinematics" model, which is generally defined in this specification as a model related to the motion and shape of an instrument, without consideration of forces on the instrument that bring about that motion. In other words, a kinematics model is based on geometric parameters and how a position of the instrument changes relative to a pre-determined or reference position or set of coordinates. The other model 222 is not a kinematics model 221 and is based on different variables. The models 221, 222 are used to control an instrument 230 or continuum manipulator, such as a guide catheter or other catheter. For ease of explanation, reference is made to a catheter 230, but it should be understood that embodiments may be utilized with various other instruments, including the catheter / sheath instrument 100 shown in Figure 1, and the same or different models 221, 222 may be used to different components as necessary.

The catheter 230 has a compliant distal end 232 that may be controllably bent and manipulated by pulling or releasing a tension element or deflection member 250, such as a pull wire, which is driven by an actuator or motor 240, such as a servo motor. For ease of explanation, reference is made to a deflection member 250 being controlled by an actuator 240. Further, Figure 2 generally illustrates a deflection element 250 integral with the catheter 230 and extending within the catheter 230, but it should be understood that the catheter 230 may include multiple deflection members 250, and that deflection members 250 may extend completely or partially through the catheter 230.

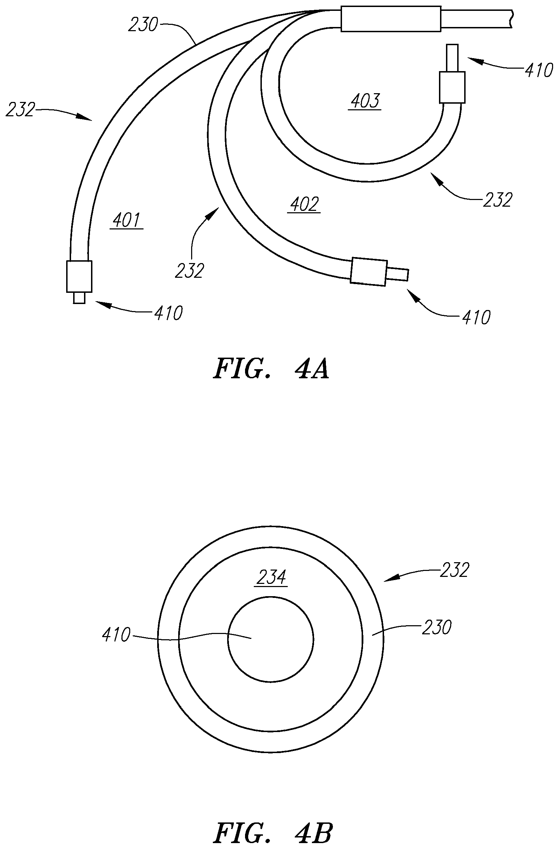

As shown in Figure 3, during use, at stage 305, the controller 210 executes the kinematics model 221, and at stage 310, executes the non-kinematics model 222. At stage 315, the actuator 240 is controlled, e.g., activated, deactivated, or adjusted, directly or indirectly, based on the outputs of these different models 221, 222. As a result, at stage 320, the deflection member 250 is controllably pulled or released by the actuator 240, thereby controllably increasing or decreasing the tension of the deflection member 250 which, in turn, results in a corresponding increase or decrease in the curvature or bending of the distal portion 232 of the catheter 230, as generally illustrated in Figures 4A-4B.

Figure 4A illustrates different degrees of bending of the distal portion 232 of the catheter 230 and a first catheter position 401 at 90 degrees, a second catheter position 402 at 180 degrees, and a third catheter position 403 at 270 degrees, which requires the largest amount of tension on the deflection member 250 relative to the other two positions 401, 402.

Figures 4A-B also illustrate a working instrument 410, one example of which is an ablation catheter, that is positioned within a lumen 234 of the catheter 230, and how different compression and bending forces at different positions 401-403 may cause the working instrument 410 to extend outwardly beyond the distal tip of the catheter 230. For example, the working instrument 410 extends outwardly beyond the distal tip of the catheter 230 at position 403 (270 degrees) to a greater degree compared to when the catheter 230 is at position 401 (90 degrees) given greater compression forces on the catheter 230 by the deflection member 250 at position 403. Embodiments advantageously account for these forces using non-kinematics models 222.

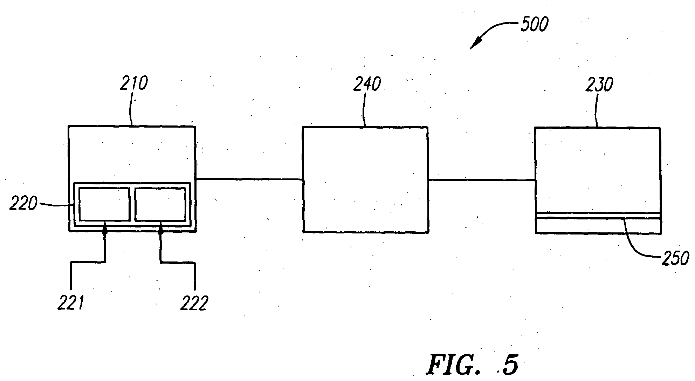

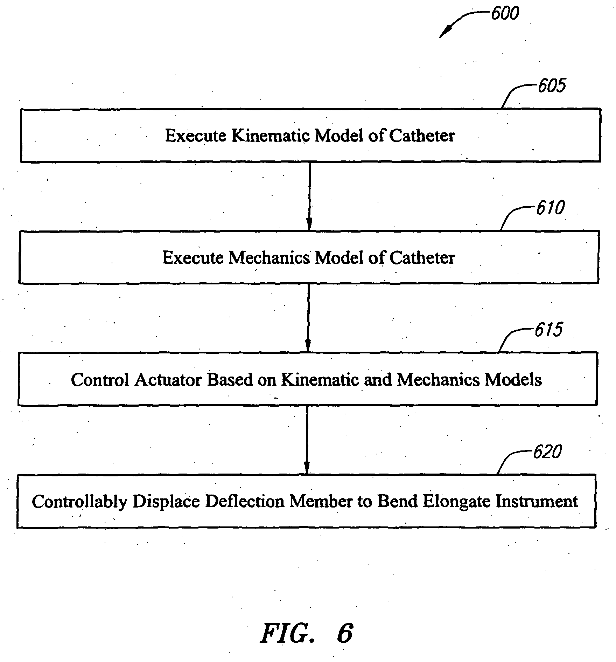

Referring to Figure 5, and with further reference to Figure 6, system 500 and method 600 embodiments of the invention advantageously provide a controller 210 that is programmed or otherwise configured with appropriate hardware and/or software to execute a control model 220 that includes a kinematics model 221 and a non-kinematics model 222 that is a mechanics model (generally referred to as mechanics model 222). A "kinematics" model 221 as used in this specification is defined as a model based on geometric attributes and shapes of the guide instrument 130 that result in the guide instrument 130 being placed in a desired position. A kinematics model may involve forward kinematics and inverse kinematics.

A "mechanics" model 222 as used in this specification is defined as a model that is based on forces applied to the catheter 230 and the resulting bending of the catheter 230. Embodiments advantageously utilize a mechanics model 222 for the inverse kinematics mapping of a catheter 230 configuration (bending and axial deflections) to displacements of deflection members 250 that are required to position the catheter 230 in particular position, e.g., represented by x,y, z coordinates. According to one example, the mechanics model 222 is a linear model. According to another embodiment, the mechanics model 222 is a static model. According to a further embodiment, the mechanics model 222 is a component of a dynamic model.

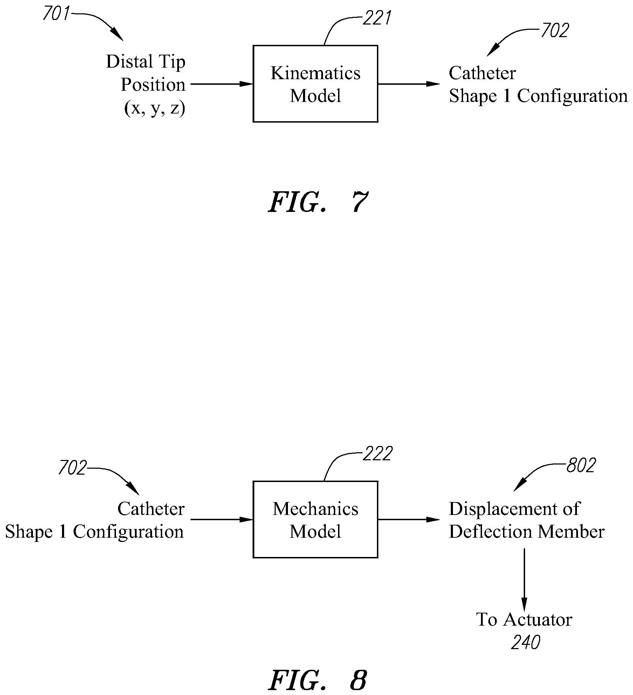

In one example, referring to Figure 7, based on inverse kinematics, an input 701 into the kinematics model 221 is a position of the distal tip of the catheter 230, represented by x-y-z coordinates, and the output 702 of the kinematics model 221 is a catheter 230 shape or configuration, as determined by corresponding actuated inputs such as pitch and yaw controls of a deflection member 250. Further aspects of a kinematics model 221 are described in U.S. Publication No. 2006/0100610 A1.

With further reference to Figure 8, in the illustrated example, the kinematics and mechanics models 221, 222 are executed serially, i.e., the kinematics model 221 is executed first, followed by the mechanics model 222. Thus, the controller 210 is configured to execute the kinematics model 221 based on the kinematics model input 701 (x,y,z position) to generate a kinematics model output 702 (catheter shape / configuration), which is then provided as an input to the mechanics model 222. The mechanics model 222 is then executed to generate a mechanics model output 802 (deflection member displacement), which is used to control the actuator 240 and move the deflection member 250 such that the distal portion 232 of the catheter 230 is bent to place the distal tip of the catheter 230 in the desired position (per the kinematics model input 701).

Further, although examples are described with reference to an inverse model and mapping a catheter 230 tip position to a corresponding deflection member 250 displacement, examples may also be implemented using a forward model and related mapping. Thus, examples are bi-directional and may be used to map x,y,z coordinates to deflection member 250 displacement, or map deflection member 250 displacement to a position in x,y,z coordinates, which may be useful for determining tip position, e.g., in the event of a fault or lost command that causes tip position to be temporary unknown.

Thus, examples advantageously utilize a kinematics model 221 that takes into account material relationships and geometric attributes of the catheter 230 to output a catheter 230 shape corresponding to a desired position, and a mechanics model 222, which considers forces on the catheter 230 resulting from manipulation of one or more deflection members 250 and the resulting compression and stretching of the compliant catheter 230, thereby advantageously providing a more complete and robust model 220 for controlling a catheter 230. examples also maintain deflection members 250 in positive tension and distribute and optimize deflection member 250 forces. Disclosed examples, therefore, address one of the challenges of known devices in accurately controlling and retaining tension in deflection members 250 that may not be the subject of the majority of the tension loading applied in a particular desired bending configuration, thereby preventing slack and lack of control over bending. Such capabilities are also particularly beneficial when, for example, the catheter carries a working instrument 410, but due to bending, the guide instrument compresses, causing the working instrument to extend outwardly beyond the distal tip of the guide instrument. Mechanics models 222 account for such forces to provide accurate positioning and driving of system components while also accounting for

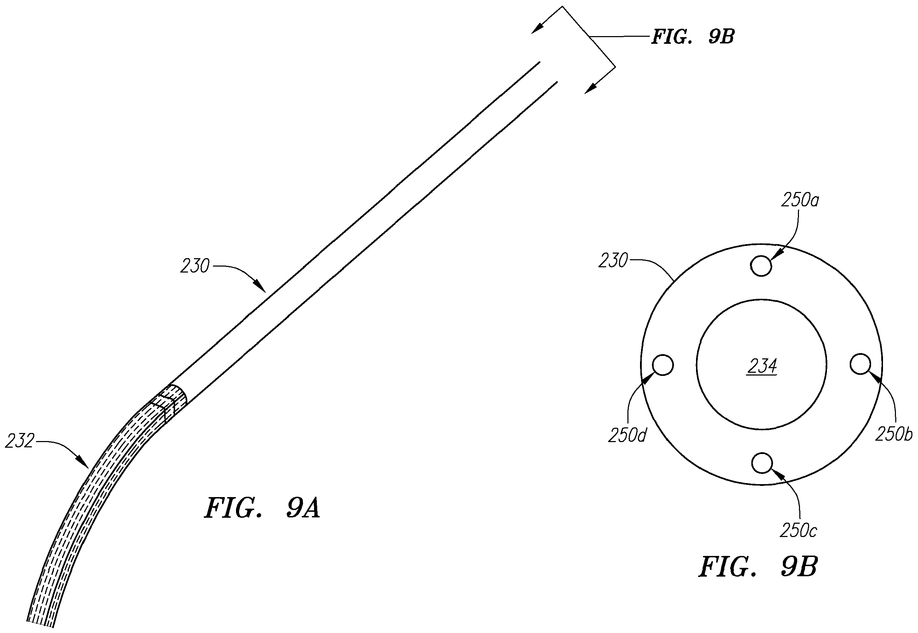

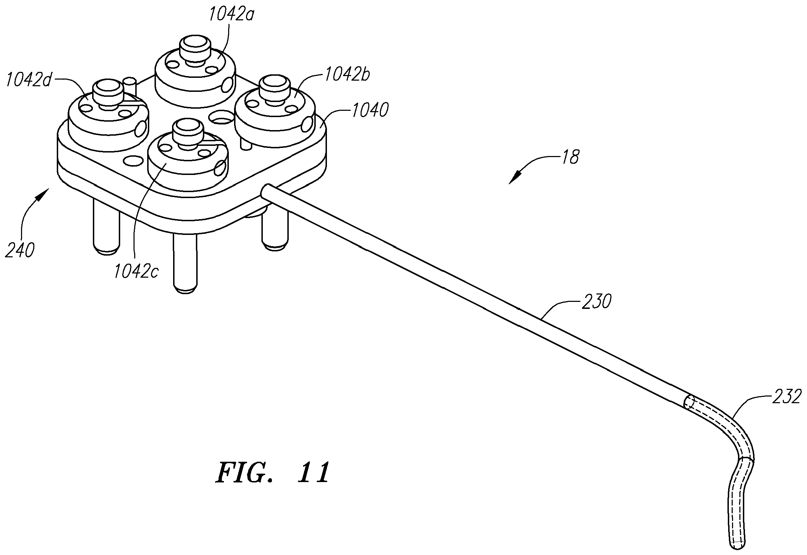

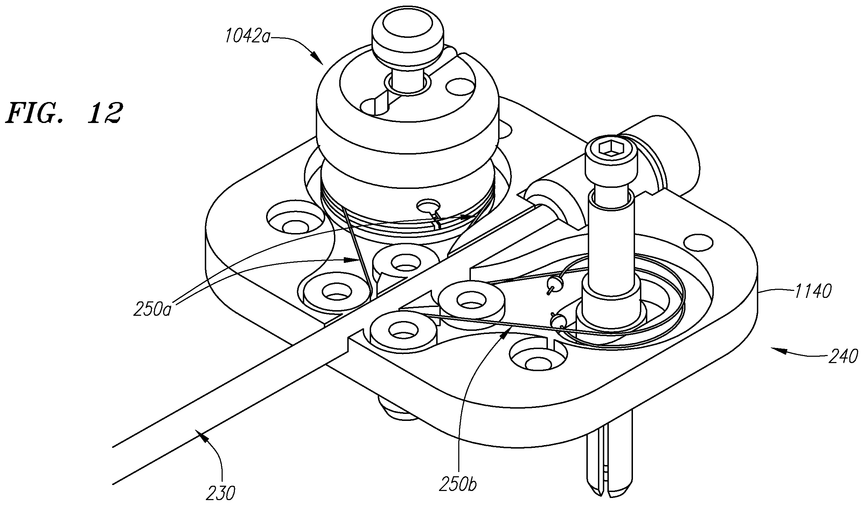

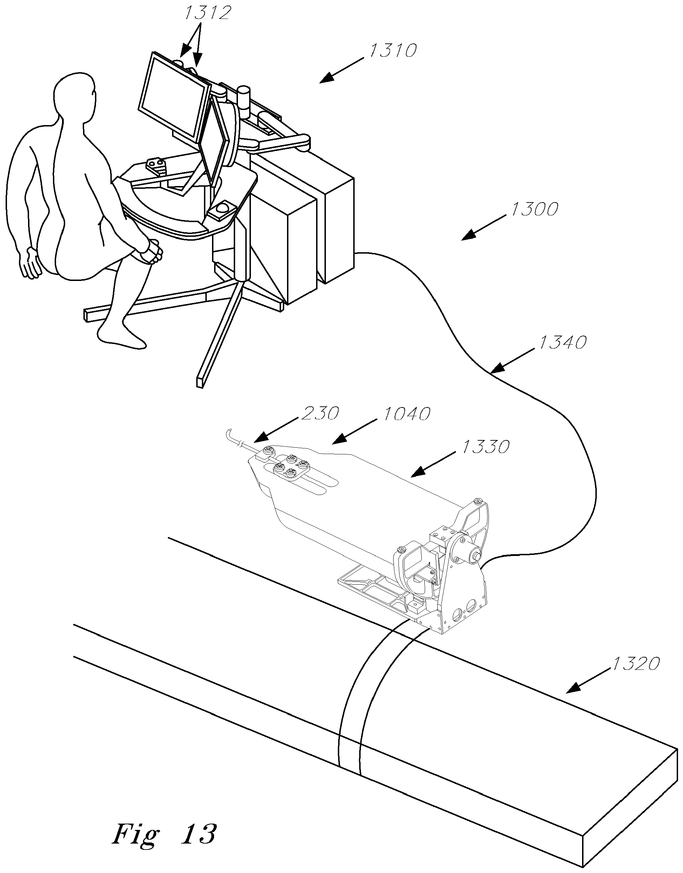

Figures 9A-13 illustrates different devices and systems that may be utilized to adjust deflection members 250 based on the mechanics model output 802 to implement the required catheter 230 movement. Referring to Figures 9A-B, the catheter 230 may define a lumen 234 for a working instrument 410 and include four deflection members 250a-d (generally 250), e.g., in the form of stainless steel pull wires. Placing tension on different deflection members 250 results in corresponding bending of the distal bending portion 232. As tension is placed only upon the top deflection member 250a, the catheter portion 232 bends upwardly. Similarly, pulling the right deflection member 250b bends the catheter portion 232 right, pulling the bottom deflection member 250c bends the catheter portion 232 downwardly, and pulling the left deflection member 250d bends the catheter portion 232 left.

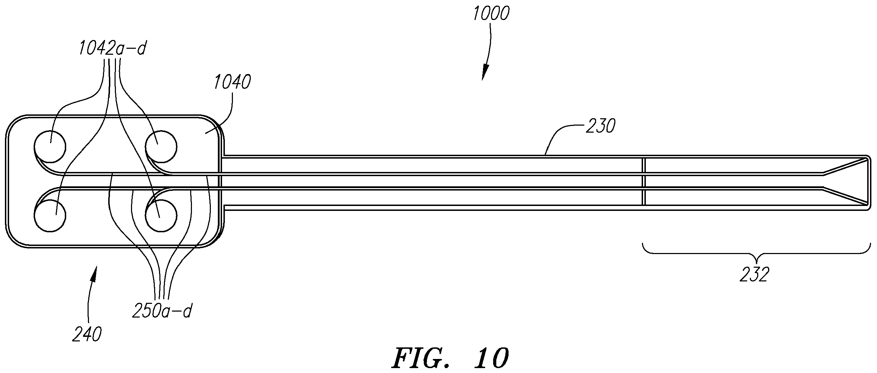

For example, the actuator 240 may be an automated instrument driver 1040 (as generally illustrated in Figures 10-12), which may include one or more servo motors that drive one or more wheels or pulleys 1042a-d around which deflection members 150 in the form of wires may be wrapped. Thus, during use, the motors may be actuated according to the control model 120, thereby rotating one or more pulleys 1042 which, in turn, adjusts the tension on deflection members 250 to impart the desired distal portion 232 bending to place the distal tip of the catheter 230 into a position corresponding to the position of the kinematics model input 701. Embodiments may be utilized within a robotic catheter system 1300, which includes an operator control station 1310 having one or more displays and/or user interfaces 1312 and an input device, e.g., in the form of a joystick, for controlling the instrument driver 1040 and catheter 230. The control station 1310 may be located remotely from an operating table 1320, to which the instrument driver 1040 and catheter 230 or other suitable instrument are coupled by a mounting brace 1330. A communication link 1340 transfers signals between the operator control station 1310 and instrument driver 1040. Further aspects of actuator and robotically controlled systems that may be used with embodiments or in which embodiments may be implemented are described in U.S. Publication No. 2006/0100610 A1.

Further, although examples are described as being utilized in various robotically controlled systems for controlling a catheter 230, e.g., with the devices and systems shown in Figures 9A-13, in other examples, a control model 220 having both kinematics and mechanics models 221, 222 may also be utilized to control a sheath instrument, e.g, the sheath 120 shown in Figure 1, in which a catheter or other suitable instrument 230 is placed. With this configuration, the sheath 120 may include deflection members 250 and a distal bending portion, and the catheter 230 is carried coaxially within, and may is movable to extend out of and retract into an opening of the sheath.

In one example, the control model including both kinematics and mechanics models 221, 222 is used to control displacement members of the catheter 230, whereas the displacement members of the sheath may be controlled using a different model, e.g., using the same or other kinematics model 221. In another embodiment, both the sheath 120 and the catheter may be controlled using kinematics and mechanics models 221, 222, and may be moved together by respective deflection members 250 under control of outputs of the same mechanics model 222 or separate, dedicated mechanics models 222.

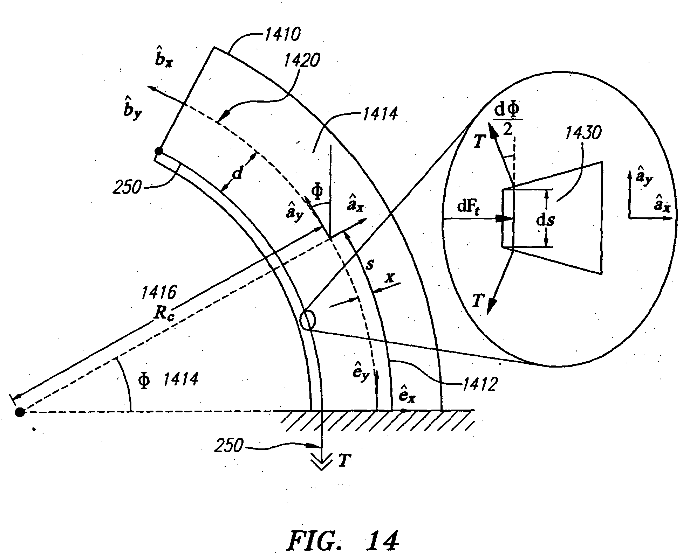

Further aspects of examples of a mechanics model 222, including the theoretical basis for a mechanics model 222 and forces and catheter attributes are utilized by the mechanics model 222 are described with reference to Figures 14-24B. Embodiments of a mechanics model are constructed according to examples based on modeling a catheter 230 as a continuum manipulator in the form of a cantilever beam, which does not have rigid links or joints, and includes a deflection member 250 or wire represented as a deflection member 250 that extends through the beam. Embodiments of a mechanics model 122 are described with reference to a single deflection member, the principles of which may be applied to a mechanics model 122 that accounts for forces on a catheter 230 resulting from multiple deflection members 250.

A similar analysis can be applied to a mechanics model 222 for a sheath 120 (e.g., as shown in Figure 1). For ease of explanation, however, reference is made to a mechanics model as applied to a catheter 230 for inverse kinematics mapping of a catheter configuration (output 702 of kinematics model 122, bending and axial mode deflections) to deflection member 250 displacements (output 802 of the mechanics model 122), while maintaining positive deflection member 250 tension.

Referring to Figure 14, a catheter 230 is represented as a planar cantilevered beam 1410 undergoing a deflection. This beam 1410 is representative of a single section of the catheter 230. The beam 1410 is subject to multiple external axial and transverse forces or loads due to actuation or pulling of the deflection member 250, which is in the form of a tendon extending through the beam 1410.

SINGLE DEFLECTION MEMBER MECHANICS

A foundation for a mechanics model 222 can be established by analysis of bending of a catheter 230 modeled as a planar cantilevered beam undergoing a deflection. This beam is representative of a single section in a catheter 230 or continuum manipulator. According to one embodiment, a mechanics model 222 is based on the premise of constant curvature beam deflection and solving for the subsequent internal loads. Given a set of material assumptions, the internal loads may be used to show consistency with the assumed circular deflection.

INTERNAL BEAM LOADING FROM SINGLE DEFLECTION MEMBER

More particularly, referring to Figure 14, a due to external loading, a beam 1410 representing a catheter 230 is deflected into a circular arc by the forces caused by deflection member 250 tension. The beam 1410 is composed of infinite concentric arcs 1412, one of which is shown to the right of the centroid 1420. Any one of these circular arcs 1412 can be described by

$$\phi = \left( \frac{1}{R_c + x} \right) s \qquad (1)$$

where s is the arc 1412 length from the $^\wedge e_x$ axis and $\phi$ is the angle 1414 between the tangent ($^\wedge a_y$) and $^\wedge e_y$, about the $^\wedge e_z$ basis vector. $R_c$ is the radius 1416 of the centroidal 1420 arc and $x$ is distance from the centroid 1420 to any other arc measured along the $^\wedge a_x$ axis. Curvature is defined as

$$\kappa := \frac{d\phi}{ds} \qquad (2)$$

and can be used to differentiate (1) yielding the curvature of a circular arc:

$$\kappa(x) = \frac{1}{R_c + x} \, .$$

(3)

When in a static configuration, each circular curve has a constant curvature $\kappa(x=c)$. A deflection member 250 is shown to exist on one of these concentric arcs of the beam 1410 specified by $\kappa_t = \kappa(-d)$ where $d$ is the distance from the deflection member 250 to the centroid 1420. The model approximates that the deflection member 250 runs parallel to the centroidal 1420 axis.

The first step in the analysis is to isolate a differential deflection member 250 segment to solve for the transverse contact force on the beam 1410. Before analyzing the deflection member 250 segment, two assumptions may be made with respect to the deflection member 250 - internally, the deflection member 250 can only resist axial tension all the way through the termination point, and externally, the deflection member 250 can only resist locally transverse loads along its length (i.e. no friction, only contact forces). While friction may be present and measurable, it is a secondary effect in terms of beam 1410 deflection. These assumptions are the equivalent of saying that the tension in the deflection member 250 is constant along its length, or that it is a pure deflection member 250. Given the premise of circular deflection geometry, a static equilibrium analysis on the differential deflection member 250 section can be analyzed.

The expanded view of a portion of Figure 14 illustrates a differential segment 1430 of the deflected deflection member 250 and beam 1410 at their contact interface in the local Frenet1 frame $a$. The deflection member 250 is shown to be an infinitesimally thin deflection member for ease of illustration. A force balance relationship can be prepared if the differential segment of the deflection member 250 is cut at the borders of the beam 1410 segment. Forces acting on the deflection member 250 are the tension $T$ at both ends (acting at an angle $d\phi/2$ from the centroidal axis), and the transverse contact force by the beam $dF_t$. The vertical components of the end tension cancel out, and the differential transverse contact force may be expressed as follows in the transverse direction:

$$dF_t = T\, d\phi$$

(4)

Dividing (4) by $ds$ results in the magnitude w of the distributed load shown in 17:

$$w \;:=\; \frac{dF_t}{ds}$$

(5)

$$= \frac{T\, d\phi}{ds}$$

(6)

$$= T \kappa_t .$$

(7)

Progression from (6) to (7) is achieved using the constant curvature of the deflection member 250 arc, expressed in (2) and (3). Given that this analysis holds for any arbitrary differential deflection member 250 section, the magnitude of the distributed force can be determined to be constant along the length of the deflection member 250 - beam 1410 interface. This result for the differential deflection member 250 segment can be transformed back into a global beam 1410 frame and integrated to determine the cumulative effect on a transverse section of the beam 1410.

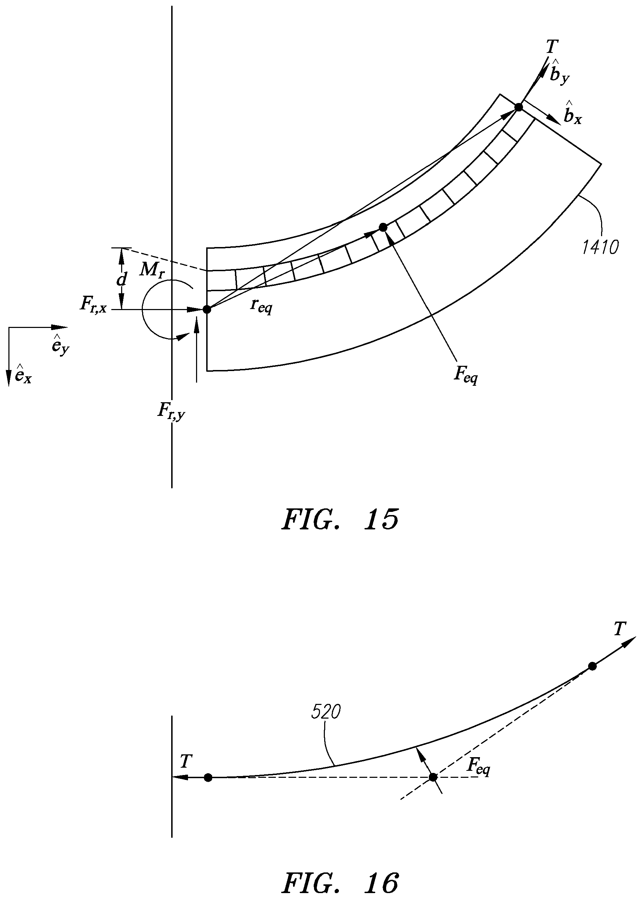

With reference to Figures 15-16, external forces acting on the beam 1410 include the deflection member 250 termination force $T$ and the distributed load $w$ from the deflection member 250 contact. The termination load acts longitudinally at the distal tip of the beam 1410, and the contact load acts transversely along the entire beam 1410 length. Calculating the internal reaction forces (both normal and shear) and the moment on the cut surface involves solving for an equivalent contact force $F_{eq}$ for the distributed load $w$. This requires integration of $w$ in the $e$ frame. The Euler rotation from the base to any transverse distal section in the $a$ frame may be expressed as

$$\mathbf{R}_a^e = \begin{bmatrix} cos\phi & -sin\phi \\ sin\phi & cos\phi \end{bmatrix} .$$

(8)

Therefore, the distributed load may be described in the e frame by

$$\mathbf{w}(s) = \mathbf{R}_a^e \left[-T\kappa_t, \ 0\right]^T \tag{9}$$

$$\mathbf{w}(s) = -\kappa_t T \left[\cos\phi, \ \sin\phi\right]^T. \tag{10}$$

Integration over the arc length provides a total equivalent contact force:

$$\mathbf{F}_{eq} = \int \mathbf{w}(s)\, ds \tag{11}$$

$$= \int -\kappa_t T \left[\cos\phi, \ \sin\phi\right]^T ds \tag{12}$$

$$= \int -\kappa_t T \left[\cos\phi, \ \sin\phi\right]^T \frac{d\phi}{\kappa_t} \tag{13}$$

$$= \int_0^{\phi_b} -T \left[\cos\phi, \ \sin\phi\right]^T d\phi \tag{14}$$

$$= T \left[-\sin\phi_b, \ \cos\phi_b - 1\right]^T \tag{15}$$

$$\tag{16}$$

The force, $F_{eq}$, is angled directly in between the $\hat{}e_x$ and $\hat{}b_x$ basis vectors. A geometry argument may be utilized to identify the point of application of $F_{eq}$. Referring to Figure 16, the point about which the moment is most easily calculated is the intersection of the end tension forces. Since it is known that $F_{eq}$ bisects the tension forces, it must also pass through this point. Therefore, $F_{eq}$ passes through the midpoint of the arc, and this may be utilized to express the force position vectors as follows:

$$\mathbf{r}_t = \left[-d - 2\sin^2\phi_b / \kappa_t, \ 2\sin\phi_b \cos\phi_b / \kappa_t\right]^T \tag{17}$$

$$\mathbf{r}_{eq} = \left[-d - \sin^2\phi_b / \kappa_t, \ \sin\phi_b \cos\phi_b / \kappa_t\right]^T. \tag{18}$$

As a result, all of the external forces and moments are defined to allow static equilibrium equations and solving of the reaction force $F_r$:

$$\sum \mathbf{F} = \mathbf{0} \tag{19}$$

$$= \mathbf{F}_{eq} + \mathbf{F}_T + \mathbf{F}_r \tag{20}$$

$$\mathbf{F}_r = -T \left[-\sin\phi_b, \ \cos\phi_b - 1\right]^T - \mathbf{R}_a^e \left[0, \ -T\right]^T \tag{21}$$

$$= \left[0, \ T\right]^T \tag{22}$$

and then solve for the reaction moment

$$\sum M \;=\; 0 \tag{23}$$

$$=\; \mathbf{r}_t \times \mathbf{F}_t + \mathbf{r}_{eq} \times \mathbf{F}_{eq} + M_r \tag{24}$$

$$M_r \;=\; -T d. \tag{25}$$

Significant results of this analysis include (22) and (25). The first element of (22) states that there is no shear force experienced on the transverse section and the second states that the longitudinal normal force is exactly the deflection member 250 tension. The moment in (25) is the deflection member 250 tension multiplied by what can be viewed as a moment arm, *d.* As shown in these expressions, there is no dependency on angle $\phi_b$, and, therefore, this result will be true for any beam 1410 articulation and for any transverse cross section along the length of the beam 1410.

DEFLECTION MEMBER TENSION TO BEAM ARTICULATION

Having determined internal loading conditions, an approach is taken from the opposite direction to demonstrate consistency with circular deflection. For this purpose, a determination is made of the resulting beam 1410 deflection assuming a cross-section of material in a cantilever beam is loaded according to (22) and (25), based on the following assumptions: 1. linear elasticity in both the axial and bending modes; 2. plain strain, meaning the material is approximately homogeneous along the longitudinal axis; 3. Saint Venant's principle applies for the internal load distribution being independent of the external load configuration; 4. planar cross sections remain plane after deflection; and material properties are symmetric about any plane containing the centroidal axis.

Since the applied moment is longitudinally invariant (25), it is known that the beam 1410 bends symmetrically about any cross-section given the above assumptions. Thus, the beam 1410 must bend in a circular arc with the curvature linearly proportional to the moment by the bending stiffness $K_b$

$$M = K_b \kappa_c \tag{26}$$

Substituting (3) and (25) into (26), the following relationship between deflection member 250 tension and curvature is obtained:

$$\kappa_c = \left(\frac{d}{K_b}\right) T \tag{27}$$

where $\kappa_c = \kappa(0)$ is the curvature of the centroid 1420. The (27) expression is significant since it states that beam 1410 curvature is controlled directly by the tension of the deflection member 250, with a gain of the moment arm to bending stiffness ratio. With this expression, it can be envisioned that pulling a deflection member 250 forces a radius of curvature (relating to the neutral surface).

Referring to Figure 16, determination of the neutral axis 1700 location may be determined using the principle of superposition to independently consider the bending and axially compressive strains. The axial ($\varepsilon_a$) and bending ($\varepsilon_b$) strains for the cross-section 1610 shown in Fig. 16 may be expressed as follows:

$$\varepsilon_b(x) \;=\; \frac{1}{K_b} M_r x \tag{28}$$

$$=\; \frac{d}{K_b} x T \tag{29}$$

$$\epsilon_a = \left(\frac{1}{K_a}\right)T. \qquad (30)$$

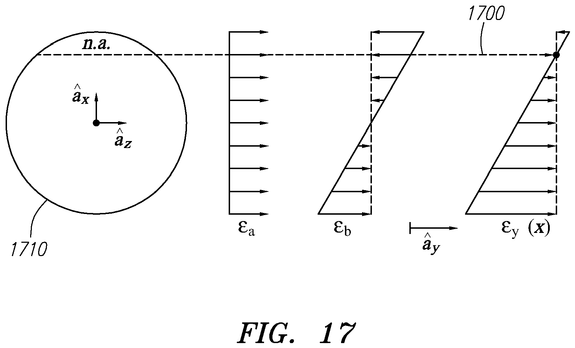

Bending strain is dependent on the distance $x$ from the centroid 1420 along the $^\wedge a_x$-axis. Summing these strain fields results in a zero point that defines the neutral axis 1700 as seen in Figure 17 and expressed as follows:

$$\epsilon_y(x) = \epsilon_b(x) + \epsilon_a . \qquad (31)$$

$$\epsilon_y(x_{na}) = 0 \qquad (32)$$

$$= \frac{d}{K_b} x_{na} T + \frac{1}{K_a} T \qquad (33)$$

$$x_{na} = \left(\frac{K_b}{K_a}\right) d^{-1}. \qquad (34)$$

Given this neutral axis 1700 location (34), under articulation of a single deflection member 250, the neutral axis 1700 is controlled by the design of the catheter 230. Design parameters that control the location of the neutral axis 1700 include the moment arm of the deflection member 250 and the bending to axial stiffness ratio.

The above description provides an expression and picture of relating deflection member 250 force to beam 1410 articulation. The tension will control the bend radius of the beam 1410 in a linear manner, and the neutral axis 1700 will be statically offset from the centroid 1420 dependent on the design of the catheter 230.

COMPARISON TO OTHER APPROACHES

Embodiments of the invention utilizing mechanics models 222 advantageously provide for more accurate modeling and control in contrast to other approaches. For example, rather than considering deflection member 250 driven continuum beam 1410 mechanics as in embodiments, another approach may be to consider either a pure bending or eccentric axial loading analysis. While this alternative may suffice for catheters 230 having very high axial to bending stiffness ratios and relatively small deflections (< 10°), traditional deflection results are obtained by integration at small angles yielding a quadratic (i.e., non-linear) expression. Such expressions become erroneous for large deflections since it is known that the curve is circular.

Embodiments utilizing a mechanics model 222 are also advantageous in the manner in which catheter 230 stiffness is considered. For example, known beam-related analyses may utilize the material quantities EI (material bending stiffness) and EA (material axial stiffness). However, embodiments of the invention are directed to a mechanics model 222 that instead utilizes $K_b$ (beam bending stiffness) and $K_a$ (beam axial stiffness). Embodiments are directed to analysis of bulk catheter 230 properties for control and, therefore, it is not necessary to consider internal material properties and geometry other than those pertaining to the assumptions stated above.

Likewise, mechanics models 222 of embodiments utilize material strain, $\varepsilon$, rather than stress, $\sigma$. This advantageously minimizes the information that must be known concerning internal structure parameters. This "black box" approach employed by embodiments is particularly beneficial since materials used for continuum instrument flexures are often composites with potentially complicated geometry, resulting in modes and numerical characteristics that may not be clearly specified. Embodiments advantageously dispose of these issues by not requiring these internal properties or parameters.

Embodiments of the invention are also designed such that the resulting control model 222 results in a working instrument 410, such as an ablation catheter, that remains undeformed axially as it bends with the catheter 230 and appears to protrude from the distal end of the catheter 230 (as shown in Figure 4A). Deflections are produced by tethering a single deflection member 250 proximally to a Chatillon force gauge while other deflection member 250s free to move.

REDUNDANT DEFLECTION MEMBER MODEL

Having described a mechanics model 222 according to one embodiment with respect to a single deflection member 250, the same mechanics model 22 principles can be extended to multiple deflection members 250. Based on the mechanics model 222 for a single deflection member 250 described above, deflection member, axial, and bending modes act as serial force transmission elements. According to one embodiment, a mechanics model 222 accounting for multiple deflection members 250 is based on the deflection members 250 acting in parallel with one another and based on superposition, which can be applied to model additional deflection members 250 since the difference among deflection members 250 is the moment arm $d_i$.

Application of model of a single deflection member 250 to multiple deflection members 250, e.g., four deflection members 250, is described based on a mechanical schematic, a matrix, and an algebraic block diagram.

DEFLECTION MEMBER DISPLACEMENTS TO BEAM CONFIGURATION

According to one embodiment, a mechanics model 222 is based on a set of expressions including a simple linear-elastic deflection member 250 model as provided below:

$$\epsilon_t = \left(\frac{1}{K_t}\right) T, \quad T \geq 0. \tag{35}$$

The inequality in (35) indicates that tension of a deflection member 250 is considered positive, and that the deflection member 250 can only experience tension. Expression (35), together with (27) and (30), demonstrate that these expressions are linear elasticity expressions having the same force but different displacements.

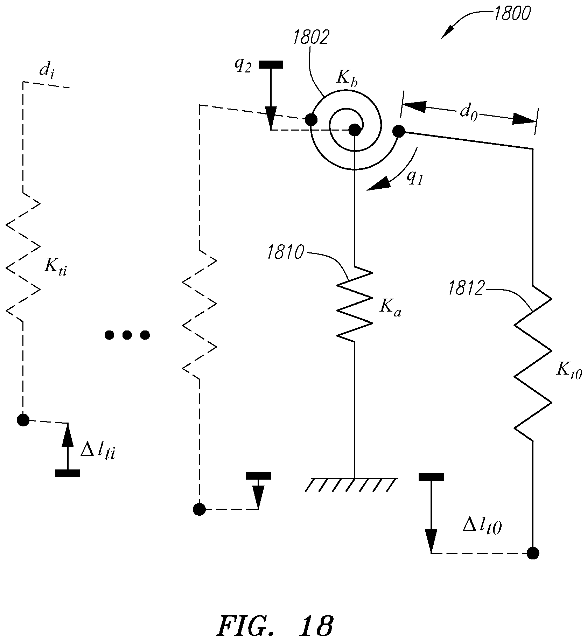

Referring to Figure 18, one manner in a mechanics model 222 of embodiments may be expressed is with an analogy to a spring model 1800 including set of series springs, which gives rise to a conceptual manipulator or catheter 230 illustrated in Figure 18 by a solid line path 1802. This spring model 1800 is composed of a rotational spring 1802 for the beam 1410 bending mode and linear displacement springs 1810 and 1812 for both the deflection member 250 and the beam's 1410 axial mode. This model 1800 provides one manner of conceptualizing the transformation from desired beam 1410 articulation, to forces, and to the required displacement of a deflection member 250. The deflection member 250 displacement, $\Delta l_t$, is not only due to its strain but its motion from ground as well. As a result, the total displacement of the deflection member 250 includes beam 1410 bending and axial compression displacements expressed as follows:

$$\Delta l_t = l_0 \left(\epsilon_b(d) + \epsilon_a + \epsilon_t\right). \tag{36}$$

The coefficient $l_0$ in (36) is the length of an undeformed beam 1410. The beam 1410 bending strain is dependent on the distance from the centroid 1420. The beam 1410 bending strain along the deflection member 250 arc may be expressed as follows by combining (26) and (28):

$$\epsilon_b(d) = \kappa_c d. \tag{37}$$

Before completing the mapping from beam 1410 articulation to deflection member 250 displacement, the inequality of (35) may be considered. If the control goal of embodiments is to enforce a desired beam 1410 curvature $\kappa_c$, then only a half axis is reachable since the deflection member 250 can only act in tension. To span the entire axis of curvature with deflection member actuators 240, a second deflection member 250 may be added to the other side of the centroid 1420. Attempting to control $m$-degrees-of-freedom with $n$-deflection members 250 may generally be expressed as

$$n >= m + 1 \tag{38}$$

The dotted lines in Figure 18 illustrate "n" deflection members 250 that may act on either side of the centroid 1420. With this model, as deflection members 250 are added, the catheter or elongate instrument 230 will be controlled by previous expressions due to superposition if the only difference is the moment arm $d_i$,

By adding deflection members 250, the entire bending axis is spanned, and when bending in one particular direction, extra degrees-of-freedom are provided if multiple deflection members 250 are in positive tension. This advantageously allows introduction of additional control capabilities. For example, referring again to Figure 16, compression and bending strain fields $\varepsilon_a$ and $\varepsilon_b(x)$ may be independently controlled.

A constraint may be expressed by specifying the strain along the centroidal 1420 axis. As discussed above, for a single deflection member 250, the neutral axis 1700 remained at a fixed location and offset from the centroid 1420 as described in (34). This implies that the material along the centroidal 1420 axis deforms as a function of curvature, and (31) may be expressed while considering multiple deflection members 250 at the centroid 1420 defined by x=0 as follows:

$$\epsilon_y(0) \; = \; \epsilon_a \tag{39}$$

$$= \; \frac{1}{K_a}\left(T_0 + T_1 + \ldots + T_n\right). \tag{40}$$

By specifying $\varepsilon_y(0)$, $x_{na}$ will no longer be fixed, it will move as necessary to satisfy (40).

For purposes of this analysis, an assumption may be made that $n$ appropriately distributed deflection members 250 are available, thus satisfying (38). This allows the beam 1410 configuration-space description to be expressed vectorially for a deflection member 250 driven catheter 230 section having a fixed initial length as follows

$$\mathbf{q} = [\kappa_c, \; \epsilon_a]^T. \tag{41}$$

Given this input, the relationship to the tension of a deflection member 250 may be expressed according to the spring model from (27) and (30) (Figure 18) as follows:

$$\begin{bmatrix} K_b & 0 \\ 0 & K_a \end{bmatrix} \begin{bmatrix} \kappa_c \\ \epsilon_a \end{bmatrix} = \begin{bmatrix} d_0 & d_1 & \ldots & d_n \\ 1 & 1 & \ldots & 1 \end{bmatrix} \begin{bmatrix} T_0 \\ T_1 \\ \vdots \\ T_n \end{bmatrix}, \tag{42}$$

This may be otherwise expressed in compact matrix form as

$$\mathbf{K}_m\,\mathbf{q} = \mathbf{G}\,\boldsymbol{\tau}.$$

In the above expression, $K_m$ is the stiffness matrix for the elongate instrument 250, $G$ is the geometry describing distributed moments and axial directed tension, and $\tau$ is the tension vector.

The last step in formulating an embodiment of a mechanics model 222 of deflection member 250 displacement as a function of beam 1410 articulation is to reintroduce the deflection member 250 displacement equation (36) vectorially and combine it with the elasticity equations (35) and (42):

$$\Delta l_t = [\Delta l_{t0}, \Delta l_{t1}, \ldots, \Delta l_{tn}]^T \tag{44}$$

$$= l_0 \begin{bmatrix} \epsilon_{b0} \\ \epsilon_{b1} \\ \vdots \\ \epsilon_{bn} \end{bmatrix} + l_0 \epsilon_a \begin{bmatrix} 1 \\ 1 \\ \vdots \\ 1 \end{bmatrix} + l_0 \begin{bmatrix} \epsilon_{t0} \\ \epsilon_{t1} \\ \vdots \\ \epsilon_{tn} \end{bmatrix} \tag{45}$$

$$= l_0 \left\{ \kappa_c \begin{bmatrix} d_0 \\ d_1 \\ \vdots \\ d_n \end{bmatrix} + \epsilon_a \begin{bmatrix} 1 \\ 1 \\ \vdots \\ 1 \end{bmatrix} + \frac{1}{K_t} \begin{bmatrix} T_0 \\ T_1 \\ \vdots \\ T_n \end{bmatrix} \right\} \tag{46}$$

leading to the following expression:

$$\Delta l_t = l_0 \left( \mathbf{G}^T + \frac{1}{K_t} \mathbf{G}^\dagger \mathbf{K}_m \right) \mathbf{q}. \tag{47}$$

A mechanics model 222 according to one embodiment is expressed in (47) above. The expression in (47) specifies how a mechanics model input in the form of a desired beam configuration (i.e., output 702 of kinematics model 121) may mapped to an associated displacement of a deflection member 250 (i.e., output 802 of mechanics model 222) for an isolated section of the catheter 230. A mechanics model 222 based on (47) is also bi-directional such that the deflection member displacement 250 (802) may be mapped to the catheter 230 shape or configuration (702). For ease of explanation, reference is made to utilizing shape or configuration as an input, to generate an output of deflection member 250 displacement $\Delta l_t$.

Mapping requires the existence of some G† (the generalized inverse of G) and

$$\tau \geq 0. \tag{48}$$

For the planar case, G† will be invertible with two or more deflection

$$\{d_i, d_j \mid d_i \neq d_j\}. \tag{49}$$

members 250:

The control strategy implemented by embodiments determines which G† to select and to ensure that (48) is satisfied.

MODEL TOPOLOGY

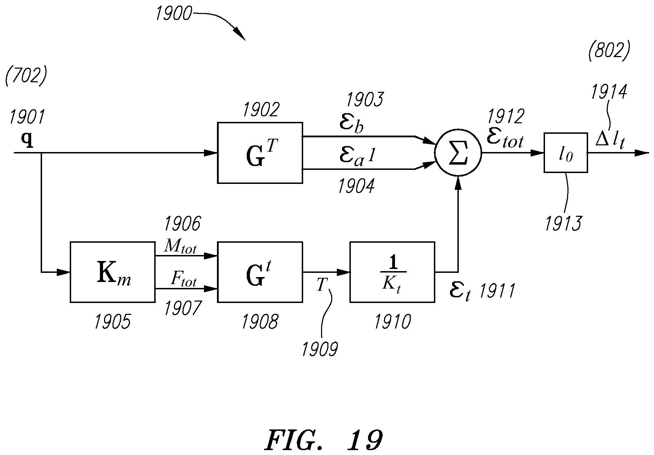

The mechanics model 222 embodiment expressed in (47) is essentially an expression for the solution to the mechanical schematic illustrated in Figure 18 and reflected as a block flow diagram 1900 in Figure 19, in which

| | | |
|---|---|---|
| q 1901 | = | output 702 of the kinematics model 221 representing a configuration or shape of the catheter 230; |
| G | = | geometric representation of the deflection member 250 in the form of a matrix; |

(continued)

| | | | |
|---|---|---|---|
| G$^T$ 1902 | = | matrix that is the transpose of matrix G, and describes the location of a deflection member 250 within the catheter 230; | |
| $\varepsilon_b$ 1903 | = | strain from the bending of the catheter 230; | |
| $\varepsilon_a$ 1904 | = | axial compression of the catheter 230; | |
| K$_m$ 1905 | = | stiffness of the catheter 230; | |
| Mtot 1906 | = | total moment on catheter 230; | |
| Ftot 1907 | = | total force on catheter 230; | |
| G$^{\dagger}$ 1908 | = | inverse of matrix G; | |
| $\tau$ 1909 | = | tension of a deflection member 250; | |
| 1/Kt 1910 | = | inverse of Kt (the stiffness of deflection member 250) | |
| $\varepsilon_t$ 1911 | = | stretching of a deflection member 250; | |
| $\varepsilon_{tot}$ 1912 | = | sum of $\varepsilon_b$ 1903, $\varepsilon_a$ 1904 and $\varepsilon_t$ 1911 | |
| I$_o$ 1912 | = | length of the deflection member 250; | |
| $\Delta$It 1913 | = | displacement of the deflection member 250 resulting from actuation of the actuator 240, i.e. output 802 of mechanics model 222, in order to place the catheter 230 distal tip at the position according to the kinematics model input 701. | |

Deflection members 250 must be displaced to account for the strains from the catheter 230 bending ($\varepsilon_b$) 1910, axial compression ($\varepsilon_a$) 1911, as well as stretching of a deflection member ($\varepsilon_t$) 1912.

More specifically, with further reference to (47), the $G^T$ block 1902 performs the kinematics transformation to bending and axial strain of the beam 1410 along the deflection member 250 arcs. The bottom portion of the model 1900 includes transformations leading to the deflection member 250 strain. First, the beam 1410 configuration is mapped to the required beam 1410 loads $M_{tot}=\sum T_i d_i$ and $F_{tot}=\sum T$. by the stiffness block $K_m$ 1903. The block G† 1906 resolves any actuation redundancy and specifies how the tensions $\tau$ of the deflection members 250 will be distributed and account for the desired beam 1410 loads. The deflection member 250 stiffness $K_t$ is then used in the inverse sense to convert the tension of a deflection member 250 to strain, $\varepsilon_t$. After all of the strains are summed together, they are multiplied by the undeformed beam length $l_0$ 1913 to form the total deflection member displacement $\Delta l_t$ 1914 which, in one embodiment, is the output 802 of the mechanics model 222.

DEFLECTION MEMBER POSITION CONTROLLER

A deflection member 250 position controller that tracks the model output is used to leverage the present mechanics model 222 relating beam 1410 configuration to displacement of one or more deflection members 250. With embodiments, a user or system element may issue a beam 1410 configuration command for a single isolated section of a catheter 230, and the controller will execute that command in real-time.

The control architecture of embodiments, including examples of limitations that may be imposed on inputs to the mechanics model that may be imposed, and then, two possible approaches to resolving deflection member redundancy and their implications on instrument performance are discussed, followed by real-time control experiments driving a catheter to verify the effectiveness and advantages of embodiments of the invention.

CONTROL ARCHITECTURE

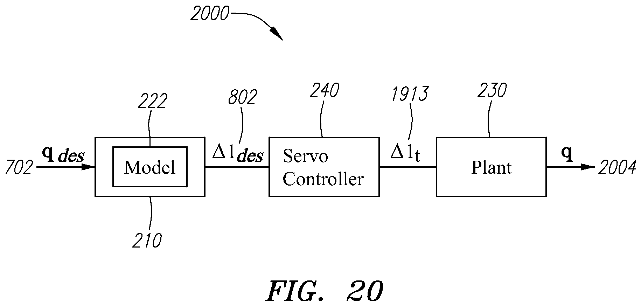

Referring to Figure 20, aspects and advantages of embodiments may be further illustrated with reference to the system 2000 block diagram, which is a block diagram representation of the expression (47) and Figure 19 constructed by a controller block 210 including the mechanics model 222, an actuator 240, e.g. servo motors, and a plant or catheter 230, which receives the output $\Delta$It 1913 (displacement of the deflection member 250), resulting in q 2004, the actual displacement or movement of the catheter 230 resulting from the kinematics model 222 output and corresponding output 1913 generated by the servo motors 240.

In the illustrated embodiment, and with further reference to Figure 20, an optional filter 2000 may be provided to filter the input to the kinematics model 222, which, in one embodiment, is $q_{des}$, or the desired beam 1410 shape or configuration of the kinematics model output 702. In one embodiment, the filter 2000 performs front-end filtering to restrict allowable inputs to the mechanics model 222. More specifically, the servo controller block 240 may include high bandwidth current amplifiers driving DC motors with encoder feedback to close the servo loop. A DC gain of one may be assumed for this block 240, and spectral separation in comparison to (unmodeled) beam mechanics such that the entire block 240 is simplified to unity gain. The plant / catheter 230 is nominally the inverse of the estimated model and, barring any significant disturbances, should approximate the desired result.

The input form from (41) can be used to specify an arbitrary duple of desired curvature and axial strain, $q_{des}$. In reality, a physical deflection member 250 driven manipulator cannot span this entire two-dimensional space. Therefore, some inputs are not attainable and should be filtered out by the filter 2100. For purposes of designing the filter 2100, an assumption may be made that achieving the desired curvature ($\kappa_{des}$) is the primary goal and that the desired axial strain ($\varepsilon_{des}$) is secondary and subject to necessary modification. The information from this filter 2100 may be used to restrict the input set a priori such that the exact input is always achievable.

For bending, if (38) is satisfied, then the manipulator or catheter 230 may arbitrarily articulate about its m axes. For axial strain, however, positive deflection member 250 tension mandates that only positive compression is possible along the centroid 1420 according to (40). This suggests that for a specified curvature, there exists a minimum axial compression $\varepsilon_{min}$. The minimum compression corresponds to the minimum total force. This occurs when the single outer-most flexor deflection member 250 bears all of the tension because it has the largest moment arm. Using G = $[d_{max}, 1]^T$ and the corresponding $\tau$ in (43) allows a determination of the minimum possible compression, thereby allowing minimization and optimization of deflection member 250 forces.

$$\varepsilon_{min}(\kappa_c) = \left(\frac{K_b}{K_a}\right)\left(\frac{1}{d_{max}}\right)\kappa_c. \tag{50}$$

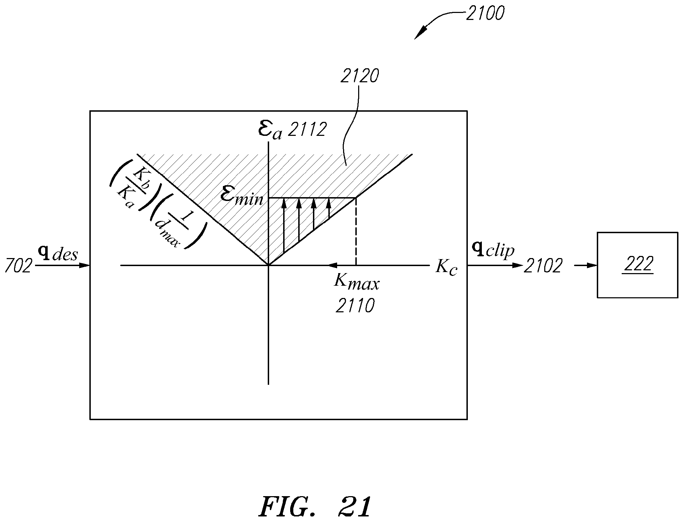

This result provides a foundation for the input filter 2100 shown in Figure 21, in which the x axis 2110 represents curvature or bending, and the y axis 2112 represents compression. If the desired input lies within the hashed subspace 2120, it may pass through unfiltered provided at least one pair of antagonistic deflection members 250 exists to generate any compression desired above the minimum. This provision can be expressed by the set $\mathbb{D}$ of a deflection member 250 pair

$$\mathbb{D} := \left\{\{d_i, d_j\} \mid (d_i \geq 0) \land (d_j \leq 0)\right\}. \tag{51}$$

However, if all of the deflection members 250 are strictly on one side of the centroid 1420, or the input axial strain is less than the minimum, then the axial strain may be clipped 2102 to a minimum. For completeness, the direction of bend may also be checked such that at least one deflection member 250 exists on the flexion side of the bend. These rules for the filter 2100 are summarized as

$$\kappa_{clip} = \begin{cases} \kappa_{des} & \text{if } sgn(\kappa_{des}) \neq sgn(d_i) \\ 0 & \text{if } sgn(\kappa_{des}) = sgn(d_i) \end{cases}$$

$$\varepsilon_{clip} = \begin{cases} \varepsilon_{des} & \text{if } \varepsilon_{des} \geq \varepsilon_{min} \text{ and } \{d_j, d_k\} \in \mathbb{D} \\ \varepsilon_{min}(\kappa_{clip}) & \text{if } \varepsilon_{des} < \varepsilon_{min} \text{ or } \{d_j, d_k\} \notin \mathbb{D} \end{cases} \tag{52}$$

where the output 2102 of the filter 2100 is expressed as $q_{clip} = [\kappa_{clip}, \varepsilon_{clip}]^T$.

Similar to the neutral axis 1700 location, the input filter 2100 of Figure 21 is dependent on the design of the manipulator or catheter 230. The bending to axial stiffness ratio is present, now in the slope of the filter 2100. If a catheter 230 is designed to have relatively high axial stiffness, for example, the slope will be very small and most inputs will be allowed to pass through the filter 2100, which is consistent with such a catheter 230 being able to bend with negligible compression, but antagonistic actuation could be used to hold the bend and arbitrarily set any positive compression. Minimal filtering would then be required other than ensuring positive axial compression and potentially antagonistic actuation.

The system shown in Figure 20 may be implemented by selecting a method for computing G† 1908 in (47). The input filter 2100 in Figure 21 and specified by (52) ensures that a feasible solution to (43) does exist (i.e. there is

some $\tau$ consistent with $q_{clip}$ where $\tau \geq 0$). However, a general inverse of $G$ does not necessarily lead to one of these feasible solutions if it does not explicitly consider the inequality constraint on $\tau$. In addition, there may be manners of inverting $G$ that may distribute the deflection member 250 forces more or less favorably. The following two sections describe a minimum-norm and minmax approach to solving for G† 1908 according to embodiments.

MINIMUM-NORM SOLUTION

To analyze the behavior of control solutions provided by embodiments, an assumption may be made that a high-level goal is to articulate a single section in bending without regard to the axial mode. One manner of implementing this is to supply a control input that maintains the axial compression constant. By selecting a specific value, information from the input filter 2100 (52) may be used to ensure that $q_{clip}=q_{des}$ and to allow the opportunity for $\tau \gtrsim 0$. This may be achieved without stressing the catheter 230 more than what is necessary by selecting the constant strain.

$$\epsilon_{des} = \epsilon_{min}(\kappa_{max}) \qquad\qquad (53)$$

where $\kappa_{max}$ is the maximum desired curvature for the specific application. This input choice is illustrated in Fig. 21 where all inputs below $\kappa_{max}$ can be increased to $\varepsilon_{min}(\kappa_{max})$ but this minimum value is not exceeded.

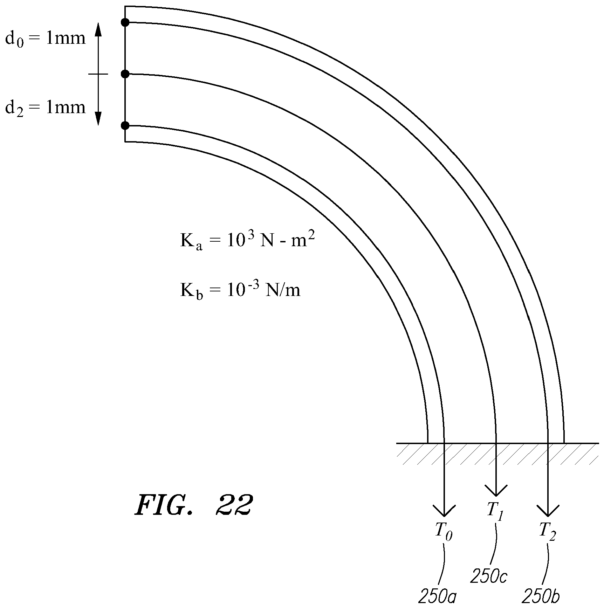

Having the control input confined to a one-dimensional space, a mathematical catheter or manipulator 230 can be constructed to simulate a simple articulation. The subject of this simulation is shown in Figure 22 with associated numerical values. The antagonistic pair of deflection members 250a-b is spread as far as possible from the centroid 1420 to reduce the minimum axial strain in (50). A third deflection member 250c is positioned along the centroid 1420 providing the redundancy used for lowering deflection member 250 forces.

This simulation utilizes the minimum-norm solution, G† = GT (G GT)-1, to solve (43) for $\tau$. There are two primary reasons to use the minimum-norm solution. For a (fat) m × n matrix where $m \leq n$, this method has a tractable, closed-form solution. The second reason is that it is generally preferable to have low deflection member 250 forces. This approach minimizes the two-norm of the deflection member 250 forces. The motivation for minimizing deflection member 250 force is because it drives a host of design requirements (i.e. motor selection, deflection member 250 yield strength, etc.). Deflection member 250 force also influences catheter 230 performance via mechanisms such as friction dependent dead-band and hysteresis.

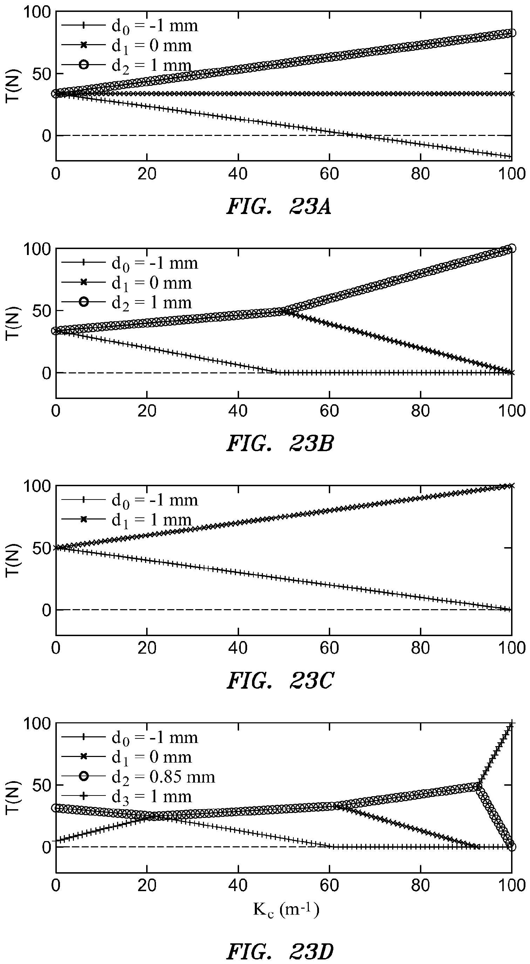

With further reference to Figure 23A, plots the results of the minimum-norm simulation described above. When the catheter 230 is oriented straight ($\kappa_c$=0), all three deflection members 250a-c are in equal tension. As the curvature of the catheter 230 is increased, the flexor deflection member increases in tension while the tension of the extensor deflection member is reduced. A zero-crossing occurs at $\kappa_c \approx 67$ and thereafter the minimum-norm solution commands a negative tension because a negative sign is inconsequential in a norm that squares values, and because the minimum-norm method does not explicitly enforce the constraint $\tau \gtrsim 0$.

Looking closely at Figure 23a indicates that at the maximum curvature, only one deflection member 250 has negative forces. Since $G \in \mathbb{R}^{2 \cdot 3}$ is full-rank, and $n$-$m$=1, there is one degree-of-freedom in selecting $\tau$. Therefore, it can be envisioned that the tension of this single deflection member 250 is increased while the tensions of other deflection members 250 adjust accordingly. In other words, it is possible to find a solution that has non-negative deflection member 250 tensions, which exists due to an initial selection of $q_{des}$ 702.

MINMAX SOLUTION

In the minimum-norm simulation of Figure 23a, the inequality constraint $\tau \gtrsim 0$ may be enforced to avoid slack deflection member 250s at high catheter 230 curvature. Furthermore, even before the zero-crossing, the deflection member 250 force distribution could be improved. Other optimization method may be used if others are not sufficient for resolving redundancy in G while maintaining required constraints.

As discussed above, reducing or minimizing deflection member 250 forces are preferable. It may also be desirable to prevent any single deflection member 250 from experiencing excessive force, which may be justifiable since both performance and design requirements are limited by the worst case of any single deflection member 250. Therefore, embodiments may utilize an alternate control objective of minimizing the maximum deflection member 250 force using only positive tensions:

$$\text{minimize} \quad \max_i(\tau_i)$$

$$\text{subject to} \quad \mathbf{K}^{-1}\mathbf{G}\tau = \mathbf{q},$$

$$\tau \geq \mathbf{0}. \tag{54}$$

In this optimization expression, the constraints are linear equalities or inequalities and the objective function is piecewise linear convex. Therefore, techniques from Linear Programming (LP) may be applied to solve for the optimal deflection member 250 forces.

Conventional LP solver routines require the problem be posed in the General Form, and expression (54) may be recast as follows:

$$\text{minimize} \quad z$$

$$\text{subject to} \quad \mathbf{K}^{-1}\mathbf{G}\tau = \mathbf{q},$$

$$\tau \geq \mathbf{0},$$

$$\tau_i \leq z. \tag{55}$$

wherein $z$ is an upper bound on any $\tau_i$. Therefore, the smallest possible value of $z$ is the maximum $\tau_i$. The constraints in (55) can be expressed in a more compact manner using an augmented decision variable $\tilde{x}$ to yield a General Form LP:

$$\text{minimize} \quad \tilde{c}^T \tilde{x}$$

$$\text{subject to} \quad \tilde{A}_{eq}\tilde{x} = \tilde{b}_{eq},$$

$$\tilde{A}\tilde{x} \leq \tilde{b} \tag{56}$$

where the augmented vectors and matrices are

$$\tilde{x} = \begin{bmatrix} \tau \\ t \end{bmatrix}, \qquad \tilde{c} = \begin{bmatrix} 0 \\ 1 \end{bmatrix},$$

$$\tilde{A} \begin{bmatrix} I & -1 \\ -I & 0 \end{bmatrix}, \qquad \tilde{b} = 0$$

$$\tilde{A}_{eq} = [\mathbf{K}^{-1}\mathbf{G}, \ 0], \qquad \tilde{b}_{eq} = \mathbf{q}.$$

Given that q has passed through the filter (52), a feasible solution to the LP problem (56) can now be determined.

Analogous to the minimum-norm simulation, a model shown in Figure 22 with the same $q_{des}$ (output of kinematics model 221) to generate the minmax solution $\tau$ plotted in Figure 23B. The first notable result is that there are no negative deflection member 250 forces. Eliminating these fictitious forces that are found in the minimum-norm solution accomplishes articulation goals.

The second key benefit of the minmax solution is found in the load distribution at smaller articulations, as seen in Figures 23A-B. When all deflection member 250s are in tension, the maximum force at a given curvature location is indeed smaller for the minmax simulation than the minimum-norm simulation.

The minmax solution may best be understood by considering how deflection member 250 loads may be re-distributed if starting from the min-norm solution in this basic three-deflection member 250 case. As an example, if the values of $\tau$ in Figure 23A at $\kappa_c$=40 are considered, since we have one-degree-of-freedom, the tension of the flexor deflection member 250 having the highest tension may be relieved. To compensate for the resulting loss in curvature, the load on the antagonistic extensor deflection member 250 may be reduced. The compression could only be maintained by then increasing the tension on the centroidal deflection member 250. Repeating this procedure, the centroidal deflection member 250 would eventually reach the force level of the flexor deflection member 250 and any further adjustment would raise it further. This is the minmax solution in Figure 23B.

From this last example, the benefit of redundant deflection members 250 becomes apparent. Without a third deflection member 250 providing redundancy, there is only one unique solution therefore no mobility. This case is demonstrated in Figure 23C in which a catheter 230 with only the two outer deflection members 250 is simulated. In this case, the maximum tension of a deflection member 250 for a given articulation is greater than the case when a redundant centroidal deflection member 250 is used for optimally distributing the loads.

The three symmetric deflection member 250 model is useful for conceptualizing results. However, the minmax solution is also capable of solving more complex models. Figure 23D illustrates how a catheter 230 having a fourth deflection member 250 is simulated. This additional deflection member 250 further advantageously minimizes the maximum force.

EXPERIMENTAL DATA

Experiments were conducted to substantiate analytical predictions achieved using mechanics model 122 embodiments. For example, to validate the combination of input filter 2100, model and redundancy resolution, a real-time controller was implemented based on the system shown in Figure 20. The catheter 230 that was used for these experiments was a four-deflection member 250 spatial manipulator that was projected onto a bend plane that cuts through two-deflection members 250. Planar projections were facilitated by introducing the constraint of zero out of plane bending moment to account for the additional variable. The minmax solution in this plane was determined by fully specifying the system with an appropriate additional constraint similar to Figure 23B. The resulting square matrix is full rank and easily invertible in real-time.

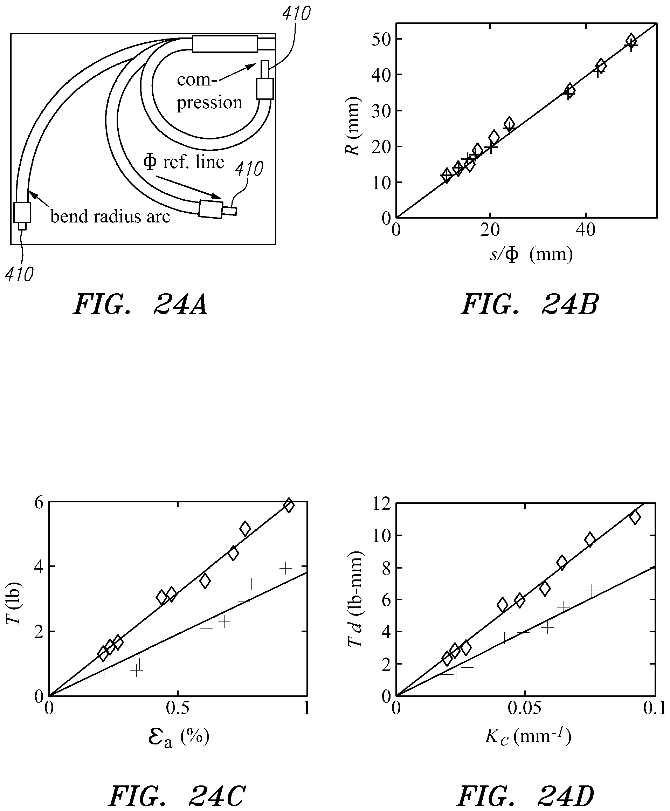

Figures 24A-D illustrates a test configuration and test data for testing involving a single deflection member 250. For this test, as shown in Figure 24A, a 3.8mm diameter catheter used during an experiment and Figures 17B-D provide the relates test data to substantiate the analytical predictions discussed above. Measurements recorded include bend radius, axial compression, and deflection member 250 tension as a function of deflection angle and initial length. The bend radius is measured by selecting a point along the catheter's 230 centroid that corresponds with an inscribed arc as seen in Figure 24A. The point selected was closest to the 180o line. Axial compression is needed to compute the net arc length and the axial strain. Axial compression is measured as the protrusion of the concentric, free-floating working instrument or element 410 that is routed down the catheter's working lumen 234. The working instrument 410 should preferably remain undeformed axially as it bends with the catheter 230 and appears to protrude outwardly from the catheter 230.

All of the deflections are produced by tethering a deflection member 250 proximally to a Chatillon force gauge while other deflection members 250 may move freely. The gauge is on a linear stage and displaced until the catheter's 230 distal tip aligns with the inscribed grid, thereby achieving a set angular deflection starting from an initially set catheter 230 length. The test deflections involved displacing a deflection member 250 to articulate the catheter 230 to 90o, 180o and 270o at three different initial lengths (60, 70 and 80-mm).

The most basic result is that of circular deflection, as shown in Figure 17A. The test results confirm that the catheter 230 that behaved according the material assumptions and mechanics model 222 discussed above.

To quantify the circular deflection, Figure 17B plots the bend radius (R) versus the arc length to deflection angle ratio ($s/\phi$). The arc length is computed as the initial undeformed catheter 230 length less the compression. From (1), this should be a line with slope one, and closely matches experimental data in which R2 = 0.95.

Referring to Figure 17C, and (27) and (30), deflection member 250 tension and axial compression can be measured in a static state. Given this data, the axial mode's force-strain relationship was plotted as shown in Figure 17C. The observed linearity substantiates (30) for which the average slope is the catheter's 230 axial stiffness $K_a$. Figure 17C illustrates the results of two tests and, therefore, includes two lines. One test approaches the set-point in flexion, and the other approaching from extension.

Figure 17D plots the moment-curvature relationship and demonstrates that curvature is linearly related to deflection member 250 tension by $K_b$, thereby validating (27) which, according to one embodiment, is the basis for a mechanics model 222.

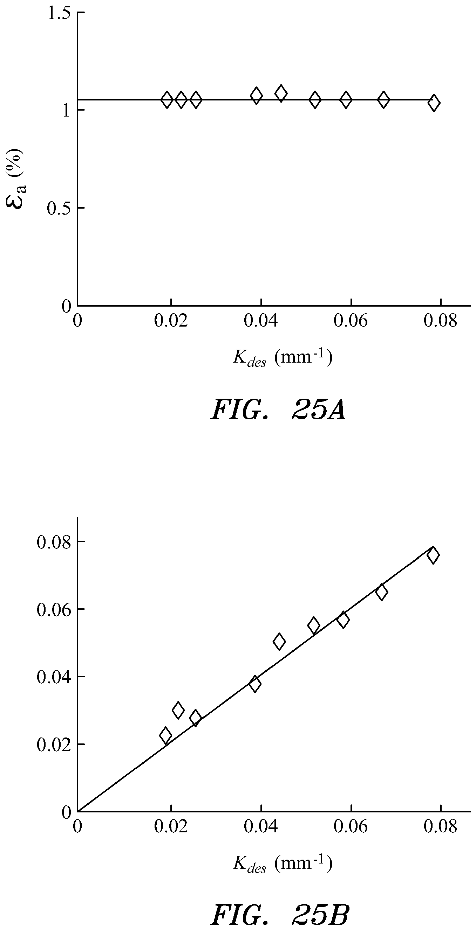

Performance was also assessed by the ability to track the desired curvature and compression in a single plane. The model parameters used were taken from articulating a catheter 230 by deflection of a single deflection member 250 as discussed above with reference to Figures 24A-D. The test data is plotted in Figures 25A-B and show fairly accurate tracking over a range of catheter 230 articulations. These results demonstrate a successful implementation of mechanics model 222 embodiments accounting for the physical constraints of actuation of the deflection member 250.

Thus, embodiments of the invention that utilize mechanics models 122 provide for the inverse kinematics mapping from beam 1410 configuration space to deflection member 250 displacement. Further, embodiments provide an understanding of which input commands are feasible given positive deflection member 250 tension, and how they might be achieved with redundant deflection members 250. The mechanics model 122, coupled with an appropriate controller, can be used to minimize occurrences of slack deflection members 250, optimize deflection member force distribution, and improve tracking accuracy.

Another application of embodiments is for use in operational space control. This could be accomplished with one additional transformation from the distal tip Cartesian coordinates to the beam configuration. When working in operational space, cascading manipulator sections serially becomes attractive for increasing end-effector mobility. Mechanics model 222 embodiments can be extended to include mechanical couplings among sections with the goal of decoupling their motion. Further, various sensor inputs and control loops may be combined with the mechanics model 222 to address internal model errors and otherwise unknown external disturbances.

Although particular embodiments have been shown and described, it should be understood that the above description is not intended to limit the scope of embodiments since various changes and modifications may be made without departing from the scope of the claims.

For example, embodiments of a mechanics model 122 are described with reference to a case of a planar, single section catheter or manipulator. This analysis, however, may serve as a foundation for higher complexity models and control systems. Further, although embodiments are primarily discussed with reference to a mechanics model 222 of an instrument 230 such as a catheter that may carry a working instrument 410, embodiments are also applicable to other instrument 230 configurations, including the instrument shown in Figure 1 that includes sheath 120 covered catheter 110. For this purpose, sheath 120 may be controlled by the same or similar kinematics model 221, mechanics model 222, or both kinematics and mechanics models 221, 222.

Thus, embodiments are intended to cover alternatives, modifications, and equivalents that fall within the scope of the claims.

**Claims**

**1.** A robotic instrument system, comprising:

a catheter instrument (230) comprising a deflection member (250); and
a controller (210) configured to control actuation of at least one servo motor (240) operatively coupled to the deflection member (250), such that the catheter instrument (230) is configured to move in response to actuation of the at least one servo motor (240), wherein the controller (210) controls positioning of the catheter instrument (230) based at least in part upon a control model (220), the control model comprising a kinematics model (221) of the catheter instrument (230);
**characterized in that**
the control model further comprises a mechanics model (222) of the catheter instrument (230), wherein the controller (210) is configured to serially execute the kinematics and mechanics models;
the controller (210) is configured to execute the kinematics model (221) based on a kinematics model input (701) to generate a kinematics model output (702), then execute the mechanics model (222) based on a mechanics model input comprising the kinematics model output (702) to generate a mechanics model output (802), the mechanics model output (802) comprising a calculated displacement of the deflection member (250); and
the controller (210) is configured to control the servo-motor (240) utilizing the calculated displacement of the deflection member (250); and
wherein the mechanics model output (802) is based at least in part on the stiffness of the catheter instrument (230) and the deflection member (250).

**2.** The system of claim 1, wherein the mechanics model (222) is a static model.

**3.** The system of claim 1, wherein the mechanics model (222) is a linear model.

**4.** The system of any of claims 1-3, wherein the control model (220) accounts for up to four deflection members (250).

**5.** The system of claim 1, wherein the kinematics model input (701) comprises a position of the catheter instrument (230), and the kinematics model output (702) comprises a configuration or shape of the catheter instrument (230) corresponding to the position.

**6.** The system of any of claims 1-5, wherein the controller (210) is configured such that the kinematics model output (702) does not directly control the servo-motor (240).

**7.** The system of any of claims 1-6, wherein the kinematics model (221) does not take into account a force on the catheter instrument (230), and wherein the mechanics model (222) takes into account a force on the catheter instrument (230).

**8.** The system of claim 1, wherein the mechanics model (222) is based on the relationship:

$$\Delta \mathbf{l}_t = l_0 \left( \mathbf{G}^T + \frac{1}{K_t} \, \mathbf{G}^\dagger \, \mathbf{K}_m \right) \mathbf{q}.$$

wherein

$\Delta$lt = a displacement of the deflection member (250) resulting from actuation of the servo-motor (240),
$l_o$ = length of the deflection member (250),
G = geometric representation of the deflection member (250) in the form of a matrix
$G^T$ = transpose of G,
$G^\dagger$ = is the inverse of G
$K_t$ = stiffness of the deflection member (250),
$K_m$ = stiffness of the catheter instrument (230), and
q= an output of the kinematics model (221) representing a configuration or shape of the catheter instrument (230).

**9.** The system of any of claims 1-8, wherein the control model (220) accounts for a curvature of the catheter instrument (230).

**10.** The system of any of claims 1-9, wherein the control model (220) accounts for a compression of the catheter instrument (230).

**11.** The system of any of claims 1-10, further comprising a filter configured to process an input to the kinematics model (221) such that the control model (220) maintains the deflection member (250) in positive tension.

**Patentansprüche**

**1.** Roboterinstrumentensystem, umfassend:

ein Katheterinstrument (230), umfassend ein Ablenkelement (250); und
ein Steuergerät (210), das dafür ausgelegt ist, die Betätigung mindestens eines Servomotors (240) zu steuern, der wirksam mit dem Ablenkelement (250) gekoppelt ist, sodass das Katheterinstrument (230) dafür ausgelegt ist, sich in Reaktion auf Betätigung des mindestens einen Servomotors (240) zu bewegen, wobei das Steuergerät (210) die Positionierung des Katheterinstruments (230) mindestens teilweise auf Basis eines Steuermodells (220) steuert, wobei das Steuermodell ein Kinematikmodell (221) des Katheterinstruments (230) umfasst;
**dadurch gekennzeichnet, dass**
das Steuermodell weiter ein Mechanikmodell (222) des Katheterinstruments (230) umfasst, wobei das Steuergerät (210) dafür ausgelegt ist, das Kinematik- und das Mechanikmodell seriell auszuführen;
das Steuergerät (210) dafür ausgelegt ist, das Kinematikmodell (221) auf Basis einer Kinematikmodelleingabe (701) auszuführen, um eine Kinematikmodellausgabe (702) zu generieren, dann das Mechanikmodell (222) auf Basis einer Mechanikmodelleingabe, die die Kinematikmodellausgabe (702) umfasst, auszuführen, um eine Mechanikmodellausgabe (802) zu generieren, wobei die Mechanikmodellausgabe (802) eine errechnete Verlagerung des Ablenkelements (250) umfasst; und
das Steuergerät (210) dafür ausgelegt ist, den Servomotor (240) unter Nutzung der errechneten Verlagerung des Ablenkelements (250) zu steuern; und
wobei die Mechanikmodellausgabe (802) mindestens teilweise auf der Steifigkeit des Katheterinstruments (230) und des Ablenkelements (250) basiert ist.

**2.** System nach Anspruch 1, wobei das Mechanikmodell (222) ein statisches Modell ist.

**3.** System nach Anspruch 1, wobei das Mechanikmodell (222) ein lineares Modell ist.

**4.** System nach einem der Ansprüche 1-3, wobei das Steuermodell (220) bis zu vier Ablenkelemente (250) berücksichtigt.

**5.** System nach Anspruch 1, wobei die Kinematikmodelleingabe (701) eine Position des Katheterinstruments (230) umfasst und die Kinematikmodellausgabe (702) eine der Position entsprechende Konfiguration oder Form des Katheterinstruments (230) umfasst.

**6.** System nach einem der Ansprüche 1-5, wobei das Steuergerät (210) so ausgelegt ist, dass die Kinematikmodellausgabe (702) den Servomotor (240) nicht direkt steuert.

**7.** System nach einem der Ansprüche 1-6, wobei das Kinematikmodell (221) eine Kraft auf dem Katheterinstrument (230) nicht berücksichtigt und wobei das Mechanikmodell (222) eine Kraft auf dem Katheterinstrument (230) berücksichtigt.

**8.** System nach Anspruch 1, wobei das Mechanikmodell (222) basiert ist auf dem Verhältnis:

$$\Delta l_t = l_0 \left( G^T + \frac{1}{K_t} G^\dagger K_m \right) q.$$

wobei

$\Delta l_t$ = eine aus der Betätigung des Servomotors (240) resultierende Verlagerung des Ablenkelements (250),
$l_0$ = Länge des Ablenkelements (250),
G = geometrische Darstellung des Ablenkelements (250) in Form einer Matrix
$G^T$ = Transponierte von G,
$G^\dagger$ = der Kehrwert von G
$K_t$ = Steifigkeit des Ablenkelements (250),
$K_m$ = Steifigkeit des Katheterinstruments (230), und
q = eine Ausgabe des Kinematikmodells (221), die eine Konfiguration oder Form des Katheterinstruments (230) darstellt.

**9.** System nach einem der Ansprüche 1-8, wobei das Steuermodell (220) eine Krümmung des Katheterinstruments (230) berücksichtigt.

**10.** System nach einem der Ansprüche 1-9, wobei das Steuermodell (220) eine Kompression des Katheterinstruments (230) berücksichtigt.

**11.** System nach einem der Ansprüche 1-10, weiter einen Filter umfassend, der dafür ausgelegt ist, eine Eingabe in das Kinematikmodell (221) so zu verarbeiten, dass das Steuermodell (220) das Ablenkelement (250) in positiver Spannung hält.

**Revendications**

**1.** Système d'instrument robotique, comprenant :

un instrument (230) faisant office de cathéter comprenant un élément de déviation (250) ; et
un dispositif de commande (210) configuré pour la commande de l'actionnement d'au moins un servomoteur (240) couplé de manière opérationnelle à l'élément de déviation (250), d'une manière telle que l'instrument (230) faisant office de cathéter est configuré pour se déplacer en réponse à un actionnement dudit au moins un servomoteur (240) ; dans lequel le dispositif de commande (210) commande le positionnement de l'instrument (230) faisant office de cathéter en se basant au moins en partie sur un modèle de commande (220), le modèle de commande comprenant un modèle cinétique (221) de l'instrument (230) faisant office de cathéter;

**caractérisé en ce que**

le module de commande comprend en outre un modèle mécanique (222) de l'instrument (230) faisant office de cathéter; dans lequel le dispositif de commande (210) est configuré pour exécuter de manière successive les modèles cinétique et mécanique ;

le dispositif de commande (210) est configuré pour exécuter le modèle cinétique (221) en se basant sur une entrée de modèle cinétique (701) dans le but de générer une sortie de modèle cinétique (702) ; pour exécuter ensuite le modèle mécanique (222) en se basant sur une entrée de modèle mécanique comprenant la sortie de modèle cinétique (702) dans le but de générer une sortie de modèle mécanique (802), la sortie de modèle mécanique (802) comprenant un déplacement calculé de l'élément de déviation (250) ; et

le dispositif de commande (210) est configuré pour la commande du servomoteur (240) en utilisant le déplacement calculé de l'élément de déviation (250) ; et

dans lequel la sortie du modèle mécanique (802) se base au moins en partie sur la rigidité de l'instrument (230) faisant office de cathéter et de l'élément de déviation (250).

2. Système selon la revendication 1, dans lequel le modèle mécanique (222) est un modèle statique.

3. Système selon la revendication 1, dans lequel le modèle mécanique (222) est un modèle linéaire.

4. Système selon l'une quelconque des revendications 1 à 3, dans lequel le modèle de commande (220) prend en compte jusqu'à quatre éléments de déviation (250).

5. Système selon la revendication 1, dans lequel l'entrée de modèle cinétique (701) comprend une position de l'instrument (230) faisant office de cathéter et la sortie de modèle cinétique (702) comprend une configuration ou une forme de l'instrument (230) faisant office de cathéter correspondant à la position.

6. Système selon l'une quelconque des revendications 1 à 5, dans lequel le dispositif de commande (210) est configuré d'une manière telle que la sortie de modèle cinétique (702) ne commande pas le servomoteur (240) d'une manière directe.

7. Système selon l'une quelconque des revendications 1 à 6, dans lequel le modèle cinétique (221) ne prend pas en compte une force s'exerçant sur l'instrument (230) faisant office de cathéter ; et dans lequel le modèle mécanique (222) prend en compte une force s'exerçant sur l'instrument (230) faisant office de cathéter.

8. Système selon la revendication 1, dans lequel le modèle mécanique (222) se base sur l'équation :

$$\Delta l_t = l_0 \left( \mathbf{G}^T + \frac{1}{K_t} \mathbf{G}^\dagger \mathbf{K}_m \right) \mathbf{q}.$$

dans laquelle

$\Delta l_t$ représente un déplacement de l'élément de déviation (250) résultant de l'actionnement du servomoteur (240) ;
$l_0$ représente la longueur de l'élément de déviation (250) ;
$G$ est une représentation géométrique de l'élément de déviation (250) sous la forme d'une matrice ;
$G^T$ représente une transposée de $G$ ;
$G^\dagger$ représente l'inverse de $G$ ;
$K_t$ représente la rigidité de l'élément de déviation (250) ;
$K_m$ représente la rigidité de l'instrument (230) faisant office de cathéter ; et
$q$ représente une sortie du modèle cinétique (221) représentant une configuration ou une forme de l'instrument (230) faisant office de cathéter.

9. Système selon l'une quelconque des revendications 1 à 8, dans lequel le module de commande (220) prend en compte une courbure de l'instrument (230) faisant office de cathéter.

**10.** Système selon l'une quelconque des revendications 1 à 9, dans lequel le module de commande (220) prend en compte une compression de l'instrument (230) faisant office de cathéter.

**11.** Système selon l'une quelconque des revendications 1 à 10, comprenant en outre un filtre configuré pour traiter une entrée dans le modèle cinétique (221) d'une manière telle que le module de commande (220) maintient l'élément de déviation (250) sous une tension positive.

*FIG. 1*

*FIG. 2*

26

300

305

Execute Kinematic Model of Catheter Control Model

310

Execute Non-Kinematic Model of Catheter Control Model

315

Control Actuator Based on Control Model

320

Controllably Displace Deflection Member to Bend Elongate Instrument

*FIG. 3*

FIG. 4A

FIG. 4B

EP 2 124 798 B1

# FIG. 5

EP 2 124 798 B1

```
                                              ┌─600

                                        ┌─605
┌──────────────────────────────────────────────┐
│        Execute Kinematic Model of Catheter     │
└──────────────────────────────────────────────┘
                        │
                        ▼                ┌─610
┌──────────────────────────────────────────────┐
│        Execute Mechanics Model of Catheter     │
└──────────────────────────────────────────────┘
                        │
                        ▼                ┌─615
┌──────────────────────────────────────────────┐
│  Control Actuator Based on Kinematic and Mechanics Models │
└──────────────────────────────────────────────┘
                        │
                        ▼                ┌─620
┌──────────────────────────────────────────────┐
│ Controllably Displace Deflection Member to Bend Elongate Instrument │
└──────────────────────────────────────────────┘
```

*FIG. 6*

30

701

Distal Tip
Position
(x, y, z)

221
Kinematics
Model

702
Catheter
Shape 1 Configuration

**FIG. 7**

702
Catheter
Shape 1 Configuration

222
Mechanics
Model

802
Displacement of
Deflection Member

To Actuator
*240*

**FIG. 8**

FIG. 9A

FIG. 9B

EP 2 124 798 B1

FIG. 10

EP 2 124 798 B1

FIG. 11

**FIG. 12**

Fig 13

**FIG. 14**

*FIG. 15*

*FIG. 16*

FIG. 17

FIG. 18

FIG. 19

2000—

$q_{des}$  222  802  240  1913  230

702 $\rightarrow$ **q** *des* $\rightarrow$ | Model | $\Delta 1$*des* | Servo Controller | $\Delta 1_t$ | Plant | **q** $\rightarrow$ 2004

210

## FIG. 20

FIG. 21

$d_0 = 1mm$

$d_2 = 1mm$

$K_a = 10^3 \, N - m^2$

$K_b = 10^{-3} \, N/m$

*FIG. 22*

$T_0$

$T_1$

$T_2$

*250a*

*250c*

*250b*

FIG. 23A

FIG. 23B

FIG. 23C

FIG. 23D

**FIG. 24A**

**FIG. 24B**

**FIG. 24C**

**FIG. 24D**

*FIG. 25A*

*FIG. 25B*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060100610 A1

- WO 2005087128 A